# EUROPEAN PATENT APPLICATION

(11) **EP 4 585 226 A1**
(43) Date of publication of application: **16.07.2025**
(21) Application number: 23863280.6
(22) Date of filing: 08.09.2023
(51) Int. Cl.: A61K 45/00, A61K 39/395, A61P 31/14, C12Q 1/6827, G01N 33/68, C07K 16/24, C07K 16/28, C12Q 1/6851

(54) **THERAPEUTIC AGENT FOR CORONAVIRUS INFECTION**

(30) Priority: 09.09.2022 JP 2022143889
(71) Applicant: INSTITUTE OF SCIENCE TOKYO, Tokyo 152-8550 (JP); Innate Cell Therapy Inc., Osaka-shi, Osaka 530-0017 (JP)
(72) Inventor: SATOH, Takashi, Tokyo 152-8550 (JP); SAKAGUCHI, Nobuo, Osaka-shi, Osaka 530-0017 (JP)
(74) Representative: Vossius & Partner Patentanwälte Rechtsanwälte mbB
(86) International application number: PCT/JP2023/032923
(87) International publication number: WO 2024/053742

(57) **Abstract**

The present invention provides a pharmaceutical composition for use in therapy or prevention of the novel coronavirus (SARS-CoV-2) disease 2019 (COVID-19), the composition containing an IL-10 inhibitor.

## Description

### TECHNICAL FIELD

The present invention relates to a method for treating or preventing infectious diseases such as the novel coronavirus disease 2019 (COVID-19), in particular to a method for suppressing the exacerbation of infectious diseases. The present invention also relates to a method for determining risk of exacerbation of infectious diseases, in particular the novel coronavirus disease 2019.

### BACKGROUND ART

The novel coronavirus disease 2019 (COVID-19) is an infectious disease caused by the causative virus, severe acute respiratory syndrome coronavirus 2 (SARS-CoV-2). This is the infectious disease that was first detected in Wuhan City, Hubei Province of the People's Republic of China on November 22nd, 2019, and since then, the numbers of infected people and deaths due to the infectious disease have continued to increase worldwide. This infectious disease is associated with acute respiratory symptoms such as fever, dry cough, and dyspnea (NPL 1), and the aggravation of the disease causes severe pneumonia and acute respiratory distress syndrome (ARDS), which is a significant problem.

SARS-CoV-2 is a positive sense single-stranded RNA virus belonging to lineage B, clade 1 of the genus Betacoronavirus. The virus binds to host cells through its trimeric spike glycoprotein composed of 2 subunits. S1 is responsible for receptor binding and S2 for membrane fusion. Angiotensin converting enzyme 2 (ACE2) is a lineage B, clade 1-specific receptor including SARS-CoV-2. The receptor binding domain (RBD) of S1 subunit directly binds to ACE2 and is therefore the most important target site to inhibit viral infection. Actually, the RBD is a common binding site for effective neutralizing antibodies identified from convalescent patients, and the study on pharmaceuticals that target the binding with ACE2 has been reported (PTL 1, and NPLs 2 and 3).

From the various clinical characteristics of severe pneumonia and acute respiratory distress syndrome (ARDS) caused after the infection with SARS-CoV-2, the immune response to the virus in the SARS-CoV-2 infected alveolus is inferred to be different from those of other types of pneumonia. As the mechanism that causes persistent alveolar inflammation, it has been proposed that alveolar macrophages infected with SARS-CoV-2 respond by producing T-cell chemoattractants, T-cells produce interferon γ to induce the release of inflammatory cytokines from the alveolar macrophages, and further promotes the activation of T cells (NPL 4).

IL-10 is an anti-inflammatory cytokine that is produced by T helper (Th) cells, macrophages, monocytes, and B cells, and is expressed as a non-covalently bound homodimer of around 37 kDa. IL-10 suppresses the secretion of proinflammatory cytokines such as TNFα, IL-1, IL-6, and IL-12, and Th1 cytokines such as IL-2 and IFNy, and controls the differentiation and proliferation of macrophages, B cells, and T cells (NPLs 5 to 8). Also, IL-10 has an ability as an inhibitor for antigen presentation, which inhibits MHCII expression and upregulation of co-stimulating molecules CD80 and CD86 (NPL 9). In addition, studies on therapy of diseases associated with IL-10 and/or IL-10 receptors (also referred to as IL-10R) have been reported (PTLs 1 and 2, and NPLs 10 to 12).

As an approach to finding a therapeutic agent to address COVID-19, analysis results using a genetic method based on data of transcriptome and proteomics of proteins that are targeted by pharmaceuticals have been reported, suggesting that the gene IFNAR2, which expresses the type-I interferon receptor, is involved in the exacerbation of COVID-19, and that the drug development that targets IFNAR2 and ACE2 should be prioritized (NPL 14).

### CITATION LIST

### PATENT LITERATURE

PTL 1: JP 6906127 B
PTL 2: JP 2011-524741 A
PTL 3: WO 2005/024027 A1

### NON PATENT LITERATURE

NPL 1: Wang, C. et al., Lancet. 2020; 395: 470-3;
NPL 2: Onodera, T. et al., 2021, Immunity 54, 2385-2398, 2021;
NPL 3: Higuchi, Y. et al., Nature Communications, 12, 3802, 2021;
NPL 4: Grant, R.A. et al., Nature, 590, 635, 2021;
NPL 5: Glocker, E.O. et al., Ann. N.Y. Acad. Sci. 1246, 102-107 (2011);
NPL 6: Moore, K.W. et al., Annu. Rev. Immunol. 19, 683-765 (2001);
NPL 7: de Waal Malefyt, R. et al., J. Exp. Med. 174, 915-924 (1991);
NPL 8: Williams, L.M. et al., Immunology 113, 281-292 (2004);
NPL 9: Mosser, D.M. et al., Immunological Reviews 226, 205-218 (2008);
NPL 10: Wang, X. et al., Cold Spring Harb Perspect Biol 2019;11:a028548;
NPL 11: Matsumoto, M. et al., Immunity 41, 1040-1051, 2014;
NPL 12: Asseman, C. et al., J Exp Med. 1999; 190(7): 995-1004;
NPL 13: Bruijnen, S.T.G. et al., Mol. Pharmaceutics 2019, 16, 273;
NPL 14: Gaziano1, L. et al., Nature Medicine, 2021, 27, 668-676

### SUMMARY OF INVENTION

### TECHNICAL PROBLEM

Severe pneumonia and acute respiratory distress syndrome (ARDS) caused after the infection with a virus such as SARS-CoV-2 have clinical characteristics different from those of other types of pneumonia. There has been demanded an effective treatment method different from existing methods, especially a treatment method for preventing exacerbation in mild patients and/or asymptomatic infected individuals. Also, there has been demanded a diagnosis method for determining the treatment for each patient infected with a virus such as SARS-CoV-2, especially a more effective method for determining risk of the exacerbation in each patient.

In one aspect of the present invention, provided is a method for treating a viral infection such as SARS-CoV-2, especially a treatment method for preventing exacerbation in mild patients and/or asymptomatic infected individuals. In another aspect of the present invention, provided is a method for determining risk of a viral infection such as SARS-CoV-2, especially a method for determining risk of the exacerbation in mild patients and/or asymptomatic infected individuals.

### SOLUTION TO PROBLEM

As a result of studies, the present inventors revealed a molecular mechanism in which IL-10 levels increase due to the upper respiratory tract infection with a virus, and the viral infection spreads to the alveolar epithelium through alveolar macrophages after the viral infection. The present inventors have therefore obtained the knowledge that a therapeutic effect on SARS-CoV-2 infection can be obtained by inhibiting the functions of IL-10 in the body, especially that the exacerbation of SARS-CoV-2 infection can be suppressed. Based on the knowledge, the present inventors have found that a therapeutic effect on SARS-CoV-2 infection can be obtained by inhibiting the functions of IL-10 in the body, especially that the exacerbation of SARS-CoV-2 infection can be suppressed. The present inventors have further found that, due to genetic polymorphism in a gene region that encodes the interferon-α receptor, a protein containing the sequences of the interferon-α receptor 2 and interleukin 10 receptor β in combination is expressed, and that the protein is involved through ACE2 expression and contributes to SARS-CoV-2 infection, and eventually achieved the present invention.

The present invention provides the following.
[1] A pharmaceutical composition for use in therapy or prevention of viral infection in which ACE2 is used as a receptor, the composition containing an IL-10 inhibitor.
[2] The pharmaceutical composition according to [1], in which the viral infection is the novel coronavirus (SARS-CoV-2) disease 2019 (COVID-19).
[3] The pharmaceutical composition according to [1] or [2], in which the IL-10 inhibitor is an anti-IL-10 monoclonal antibody, an anti-IL-10 receptor monoclonal antibody, or an antigen-binding fragment thereof.
[4] The pharmaceutical composition according to any of [1] to [3], in which the IL-10 inhibitor is an anti-IL-10 receptor β monoclonal antibody or an antigen-binding fragment thereof.
[5] The pharmaceutical composition according to any of [1] to [4], for use in therapy or prevention of exacerbation of COVID-19.
[6] The pharmaceutical composition according to any of [1] to [5], in which the antibody or antigen-binding fragment is a full-length antibody, Fab, Fab', F(ab')₂, Fv, scFv, dsFv, a diabody, or sc(Fv)₂.
[7] The pharmaceutical composition according to any of [1] to [6], in which the IL-10 inhibitor is a mouse antibody, a humanized antibody, a human antibody, a chimeric antibody, or an antigen-binding fragment thereof.
[8] The pharmaceutical composition according to any one of [1] to [7], for use in therapy or prevention of exacerbation of COVID-19 accompanied by pneumonia.
[9] The pharmaceutical composition according to any of [1] to [8], for administration to a subject who has a measured blood concentration of IL-10 that is equal to or more than a predetermined value.
[10] The pharmaceutical composition according to any of [1] to [9], for administration to a subject who has been confirmed to have a measured expression level of a protein (CIDRE) that is equal to or more than a predetermined level, the protein having all or a part of the amino acid sequence of an interferon-α receptor 2 and all or a part of the amino acid sequence of an IL-10 receptor β.
[11] The pharmaceutical composition according to any one of [1] to [10], for administration to a subject who has been determined to be expected to have a therapeutic or preventive effect of administration of the IL-10 inhibitor, or a subject who has been determined to have a high risk of exacerbation of the novel coronavirus (SARS-CoV-2) disease 2019 (COVID-19).
[12] The pharmaceutical composition according to any of [1] to [11], for administration to a subject who has been determined to be a subject of administration, in which
   the above determination is performed based on:
   1) measurement of the blood concentration of IL-10 in the subject;
   2) measurement of the expression level of the IL-10 receptor in the subject;
   3) quantification of chimeric transcripts of an IL10RB gene and an IFNAR2 gene;
   4) measurement of the expression level of the protein (CIDRE) having all or a part of the amino acid sequence of the interferon-α receptor 2 and all or a part of the amino acid sequence of the IL-10 receptor β; or
   5) presence or absence of mutations in a genomic region that controls alternative splicing of the IL10RB gene and/or the IFNAR2 gene.
[13] The pharmaceutical composition according to [12], in which the subject is determined to be a subject of administration when the blood concentration of IL-10 in the subject is equal to or more than the predetermined value.
[14] The pharmaceutical composition according to [12], in which the subject is determined to be a subject of administration when the expression level of the IL-10 receptor in the subject is equal to or more than the predetermined level.
[15] The pharmaceutical composition according to [12], in which the subject is determined to be a subject of administration when an amount of chimeric transcription products of the IL10RB gene and the IFNAR2 gene in the subject is equal to or more than the predetermined amount.
[16] The pharmaceutical composition according to [12], in which the subject is determined to be a subject of administration when the expression level of CIDRE is equal to or more than the predetermined level.
[17] The pharmaceutical composition according to [12], in which the subject is determined to be a subject of administration when a mutation is present in the genomic region that controls alternative splicing of the IL10RB gene.
[18] The pharmaceutical composition according to [12], in which the subject is determined to be a subject of administration when a mutation is present at rs13050728.
[19] The pharmaceutical composition according to any one of [1] to [18], in which the subject has a past history of lung disease.
[20] A method for determining risk of exacerbation of the novel coronavirus (SARS-CoV-2) disease 2019 (COVID-19), in which
   the above determination includes:
   1) measurement of the blood concentration of IL-10 in the subject;
   2) measurement of the expression level of the IL-10 receptor in the subject;
   3) quantification of chimeric transcripts of an IL10RB gene and an IFNAR2 gene;
   4) measurement of the expression level of the protein (CIDRE) having all or a part of the amino acid sequence of the interferon-α receptor 2 and all or a part of the amino acid sequence of the IL-10 receptor β; or
   5) confirmation of the presence or absence of mutations in a genomic region that controls alternative splicing of the IL10RB gene and/or the IFNAR2 gene.
[21] The method according to [20], further including determining that the subject has a high risk of the exacerbation when the blood concentration of IL-10 in the subject is equal to or more than the predetermined value.
[22] The method according to [20], further including determining that the subject has a high risk of the exacerbation when the expression level of the IL-10 receptor in the subject is equal to or more than the predetermined level.
[23] The method according to [20], further including determining that the subject has a high risk of the exacerbation when the amount of chimeric transcripts of the IL10RB gene and the IFNAR2 gene are equal to or more than the predetermined amount.
[24] The method according to [20], further including determining that the subject has a high risk of the exacerbation when the expression level of CIDRE is equal to or more than a predetermined level.
[25] The method according to [20], further including determining that a mutation is present in the genomic region that controls alternative splicing of the IL10RB gene, and the chimeric transcripts of the IL10RB gene and the IFNAR2 gene are detected in the subject.
[26] The method according to [20], further including determining that a mutation is present at rs13050728, and the chimeric transcripts of the IL10RB gene and the IFNAR2 gene are detected in the subject.
[27] The method according to any one of [20] to [26], in which the determination is performed using a blood sample collected from the subject.
[28] The method according to any of [20] to [27], in which the subject is not infected with SARS-CoV-2.

### ADVANTAGEOUS EFFECTS OF INVENTION

The present invention provides a pharmaceutical composition for use in therapy or prevention of the novel coronavirus (SARS-CoV-2) disease 2019 (COVID-19), especially a pharmaceutical composition suitable for use in therapy or prevention of exacerbation of COVID-19.

### BRIEF DESCRIPTION OF DRAWINGS

[Fig. 1] Fig. 1 is a volcano plot showing differentially expressed genes (DEGs) in the lungs by comparing uninfected (after 0 days) and 5 days after infection, in an in vivo infection experiment of Test Example 1.
[Fig. 2] Fig. 2 is a Venn plot showing genes significantly associated with "cell death", "autophagy", "immune response", "cytokine production", and "myeloid leukocyte activation", by selecting a total of 5009 genes with Log₂FC exceeding 2 in Fig. 1, analyzing them using g:Profiler, and selecting from gene ontology (GO).
[Fig. 3-1] Fig. 3 is graphs showing IL-10 blood concentrations of patients with exacerbation (n=27) and patients without exacerbation (n=93) among 128 COVID-19 patients. The IL-10 blood concentrations in the patients with exacerbation were confirmed to be significantly higher than those in the patients without exacerbation (p<0.005).
[Fig. 3-2] Fig. 3 is graphs showing IL-10 blood concentrations of patients with exacerbation (n=27) and patients without exacerbation (n=93) among 128 COVID-19 patients. The IL-10 blood concentrations in the patients with exacerbation were confirmed to be significantly higher than those in the patients without exacerbation (p<0.005).
[Fig. 4] Fig. 4 is a graph showing the influence of IL-10 on the mRNA expression level of ACE2 in alveolar macrophages infected with SARS-CoV-2 in Test Example 3. The mRNA expression levels of ACE2 were measured by PCR (qPCR).
[Fig. 5] Fig. 5 is graphs showing the influence of IL-10 on the mRNA expression levels of ACE2 in cells infected with α strain, β strain, γ strain, and Omicron strain of SARS-CoV-2 in the same manner as the test which results are shown in Fig. 4.
[Fig. 6] Fig. 6 is graphs showing analysis results of flow cytometry (FACS) in Test Example 4. The analysis results show that the expression levels of ACE2 increased by the addition of IL-10.
[Fig. 7] Fig. 7 is photographs showing results of confirming the ACE expression in the virus-infected cells of Test Example 3 by an immunostaining method.
[Fig. 8] Fig. 8 is graphs showing measurement results of the expression levels of inflammatory cytokines in Bal cells of golden hamsters infected with SARS-CoV-2.
[Fig. 9] Fig. 9 is a graph showing correlation of mutations (C/C, T/C, and T/T) at rs13050728 in the IL10RB genes with changes in splicing pattern.
[Fig. 10] Fig. 10 is a graph showing results of FACS analysis of the CIDRE-expressing macrophage cell line constructed in Test Example 8.
[Fig. 11] Fig. 11 is a graph showing measurement results of CIDRE expression level in the CIDRE-expressing macrophage cell line constructed in Test Example 8.
[Fig. 12] Fig. 12 is a graph showing results of FACS analysis of the CIDRE-expressing macrophage cell line constructed in Test Example 8, after stimulation with IL-10 and IFNα for 48 hours.
[Fig. 13] Fig. 13 is a graph showing the measurement results of the mRNA expression level of ACE2 in the alveolar macrophages infected with SARS-CoV-2. The results show that the expression level of ACE2 increases by the addition of IL-10, whereas the expression level decreases by the addition of an anti-IL10R antibody.
[Fig. 14] Fig. 14 is a diagram showing results of Western blotting for confirming the production of phosphorylated stat3 in human CIDRE-expressing alveolar macrophages.
[Fig. 15] Fig. 15 is a diagram showing results of Western blotting for confirming the production of phosphorylated stat3 in human CIDRE-expressing alveolar macrophages.
[Fig. 16] Fig. 16 is diagrams showing FACS analysis results for confirming the binding of IFNα in human CIDRE-expressing cells.
[Fig. 17] Fig. 17 is graphs showing results of colocalization analysis of signals obtained by sQTL and GWAS for COVID-19 exacerbation.
[Fig. 18] Fig. 18 is a graph showing test results for confirming the correlation between the presence or absence of T/T mutation and the CIDRE expression level, using total RNA in human-derived cells.
[Fig. 19] Fig. 19 is a diagram showing results of Western blotting for confirming the size of CIDRE in comparison with IL-10Rb.

### DESCRIPTION OF EMBODIMENTS

Viral infection in which ACE2 is used as a receptor used herein is not particularly limited as long as it is viral infection in which binding of the virus to ACE2 is responsible for the infection. In one aspect of the present invention, the virus binds to ACE2 that is expressed on a cell surface as a ligand and is taken into cells. In one embodiment, the cell is an immune cell, for example, a macrophage, and specifically an alveolar macrophage. In one embodiment, the viral infection is COVID-19, and the virus is SARS-CoV-2.

The IL-10 inhibitor in the present invention is not particularly limited, and as long as it is a substance that inhibits in vivo reactions or signaling through IL-10, it is not particularly limited. In one aspect of the present invention, the IL-10 inhibitor may be a peptide, a protein, a nucleic acid, or a low molecular weight compound. In one embodiment, the IL-10 inhibiting agent is selected from an anti-IL-10 antibody, an anti-IL-10 receptor antibody, a low molecular weight compound having antagonistic activity on the IL-10 receptor, a polynucleotide that inhibits the expression of IL-10, and a polynucleotide that inhibits the expression of the IL-10 receptor. In a further embodiment, the IL-10 inhibitor is an anti-IL-10 antibody, especially an anti-IL-10 antibody having neutralizing activity of IL-10, or an anti-IL-10 receptor antibody, especially an anti-IL-10 receptor antibody having antagonistic activity of IL-10 receptor.

According to one aspect of the present invention, anti-IL-10 antibodies can be used as the IL-10 inhibitor. The anti-IL-10 antibody can be prepared by a method known to those skilled in the art. Also, the anti-IL-10 antibody can be obtained by purchase. The anti-IL-10 antibody can be selected, for example, from commercially available antibodies such as Clone 23738 (R&D Systems, Inc.), sc-8438 (SANTA CRUZ BIOTECHNOLOGY, INC.), JES3-12G8 (BioLegend, Inc.), JES3-19F1 (BioLegend, Inc.), JES3-19F1 (RUO) (BD Biosciences), JES5-16E3 (Invitrogen), ab259402 (Abcam) and variants thereof.

According to one aspect of the present invention, anti-IL-10 receptors (IL-10R) can be used as the IL-10 inhibitor. IL-10R consists of two subunits: IL-10Rα (IL-10R alpha or IL-10R1, CD210) and IL-10Rβ (IL-10R beta, IL-10R2), which are members of the class II cytokine receptor family. Without wishing to be bound by theory, binding of IL-10 to a heterodimeric IL-10R is thought to result in the activation of receptor-associated Jak1 and Tyk2 protein tyrosin kinases, and subsequent tyrosine phosphorylation and activation of DNA binding of signal transducer and activator of transcription 3 (STAT3) and STAT1.

IL-10Rα is a ligand binding subunit, and binds to IL-10 with high affinity (Kd of about 35 to 200 pM). Human IL-10Rα contains 578 amino acids with a molecular size of 90 to 110 kDa, and has 60% homology with mouse IL-10Rα. According to one aspect of the present invention, anti-IL-10 receptor α (IL-10Rα) antibodies can be used as the IL-10 inhibitor. IL-10Rβ contributes little to IL-10 binding affinity, and it is thought to be an accessory subunit of IL-10R for signaling.

The binding sites of human IL-10 and human IL-10Rα are known to have been mapped and discontinuous. One neutralizing anti-IL-10Rα monoclonal antibody (#MAB274, clone 37607, R&D Systems, Inc.) has been reported to recognize discontinuous epitopes that overlap with some of the IL-10/IL-10R binding sites. It is revealed (NPL 24), which suggests that the natural conformation of IL-10 may be important for the generation of neutralizing antibody. In one aspect of the present invention, commercially available neutralizing anti-IL-10Rα monoclonal antibodies can be used as the IL-10 inhibitor. Examples of commercially available antibodies include antibodies 3F9 (#308806, BioLegend, Inc.), SPM466 (#E8574, Spring Biosciences, Inc.), and 37607 (#MAB274, R&D Systems, Inc.).

In one aspect of the present invention, neutralizing anti-IL-10Rβ monoclonal antibodies can be used as the IL-10 inhibitor.

The anti-IL10Rα monoclonal antibodies described in the present invention encompass anti-human IL-10Rα monoclonal antibodies. Examples of the anti-human IL-10R α antibodies disclosed herein include 136C5 described in PTL 2 (antibody-producing hybridoma deposited on April 8, 2008, with deposit number of PTA-9131, ATCC 10801 University Blvd., Manassas, VA 20110-2209), 136C8 (antibody-producing hybridoma deposited on April 8, 2008 with deposit number of PTA-9132, ATCC 10801 University Blvd., Manassas, VA 20110-2209), and 136D29 (antibody-producing hybridoma deposited on April 8, 2008 with deposit number of PTA-9133, ATCC 10801 University Blvd., Manassas, VA 20110-2209).

Examples of type I interferons include interferon α (IFNα), interferon β (IFNβ), interferon ε (IFNε), interferon κ (IFNκ), and interferon ω (IFNω). In one embodiment of the present invention, examples of type I interferons include interferon α (IFNα), and interferon β (IFNβ).

According to one aspect of the present invention, the pharmaceutical composition can be used in prevention or therapy of lung diseases, especially pneumonia. According to one aspect of the present invention, the pharmaceutical composition can be used in prevention or therapy of diseases in organs other than the lung, for example, the heart, kidney, respiratory system, blood, gastrointestinal system, skin, or nervous system, especially inflammatory diseases. According to one aspect of the present invention, the pharmaceutical composition can be used in prevention or therapy of multisystem inflammatory syndrome or adult multisystem inflammatory syndrome as a complication after the onset of COVID-19.

When the IL-10 inhibitor is a nucleic acid, it is not particularly limited as long as the nucleic acid inhibits signals of IL-10/IL-10R or type I IFN/IFNAR. In one aspect of the present invention, the IL-10 inhibitor is a nucleic acid selected from an antisense, siRNA, miRNA, a decoy, an aptamer, or CpG oligo. In one embodiment of the present invention, when the IL-10 inhibitor is a nucleic acid, the nucleic acid inhibits the expression of IL-10, IL-10Rα, or IL-10RNAβ.

The term "antibody" as used herein refers to a protein that binds to other molecules (antigens) through the heavy and light chain variable domains, V_{H} and V_{L}, respectively. The antibody includes a full-length antibody containing two heavy chain and two light chain sequences. The antibody can have kappa or lambda light chain sequences in full length, such as naturally occurring antibodies, in a mixture thereof (i.e., fusion of kappa and lambda chain sequences), and in a subsequence/fragment thereof. Naturally occurring antibody molecules have two kappa or two lambda light chains.

The antibody includes monoclonal or polyclonal immunoglobulin molecules that belong to any class such as IgM, IgG, IgA, IgE, and IgD, and any subclass thereof. Typical subclasses of IgG are IgG₁, IgG₂, IgG₃, and IgG₄. The "monoclonal" antibody refers to an antibody based on, obtained by, or derived from a single clone, containing any eukaryotic, prokaryotic, or phage clone. The "monoclonal" antibody is therefore defined by its structure, not by the method by which it is produced.

In one aspect of the present invention, the antibody of the present invention is an isolated antibody. The isolated antibody does not include a naturally occurring antibody without any external manipulation (artificial manipulation), in other words, an antibody that are produced in an individual body and remains there. The antibody of the present invention is typically a monoclonal antibody, or an antigen-binding fragment thereof.

As used herein, the term "binding" or "bind" when used in reference to an antibody, refers to the interaction of the antibody or a subsequence thereof with a corresponding epitope (antigenic determinant) on an antigen at the molecular level. Specific and selective binding can be distinguished from non-specific binding using an assay known in the art (e.g., immunoprecipitation, ELISA, flow cytometry, or Western blotting).

Epitopes are generally short amino acid sequences, for example, about 5 to 15 amino acids in length. Epitopes may be contiguous or non-contiguous. An epitope consisting of non-contiguous amino acid sequences is generated due to protein folding. For example, an epitope can include non-contiguous amino acid sequences, such as 5 amino acid sequences and 8 amino acid sequences, which are not contiguous with each other, but form an epitope due to protein folding. Techniques for identifying epitopes are known to those skilled in the art, and include screening overlapping oligopeptides for binding to an antibody (e.g., U.S. Patent No. 4,708,871), a phage display peptide library kit, which is commercially available for epitope mapping (New England BioLabs, Inc.). Epitopes may also be identified by inference when a peptide sequence of the length of the epitope is used to immunize animals from which antibodies that bind to the peptide sequence are obtained and when imparted, and by be predicted using computer programs, such as BEPITOPE (Odorico et al., J. Mol. Recognit. 16:20 (2003)).

The term "isolated" as used herein as a modifier of a composition (e.g., an antibody, a subsequence, a modified form, and a nucleic acid that encodes the same), means that the composition is artificially made, or separated, completely or at least partially, from its naturally occurring in vivo environment. Generally, isolated compositions are substantially free of one or more substances with which normally they are naturally associated, for example, one or more proteins, nucleic acids, lipids, carbohydrates, and/or cell membranes. The term "isolated" does not exclude alternative physical forms of the composition, such as a fusion/chimera, multimer/oligomer, modification (e.g., phosphorylated, glycosylated, and lipidated), derivative, or form expressed in host cells artificially produced.

The antibody used herein encompasses mammalian, primatized, humanized, and fully human antibodies and chimeras. The mammalian antibody is an antibody produced by a transgenic or non-transgenic mammal, or a non-mammalian organism modified to produce a mammalian antibody, such as a non-mammalian cell (bacterium, yeast, insect cell), animal, or plant.

The term "human" as used herein in reference to an antibody, means that the amino acid sequence of the antibody is a fully human amino acid sequence, in other words, human heavy and human light chain variable and human constant regions. Accordingly, all the amino acids are human amino acids or are present in human antibodies. Antibodies that are non-human antibodies can be prepared as fully human antibodies by substituting non-human amino acid residues with the amino acid residues present in human antibodies. The amino acid residues present in human antibodies, CDR region maps, and human antibody consensus residues are known in the art (see, e.g., Kabat, Sequences of Proteins of Immunological Interest, 4th Ed. US Department of Health and Human Services. Public Health Service

(1987); Chothia and Lesk (1987)). A consensus sequence of human V_{H} subgroup III, based on a survey of 22 known human V_{H} III sequences, and a consensus sequence of human V_{L} kappa-chain subgroup I, based on a survey of 30 known human kappa I sequences are described in Padlan Mol. Immunol. 31:169 (1994); and Padlan Mol. Immunol. 28:489 (1991). Human antibodies therefore include antibodies in which one or more amino acid residues are substituted with one or more amino acids present in any other human antibody.

The term "humanized" as used herein in reference to an antibody, means that the amino acid sequence of the antibody has non-human amino acid residues (e.g., mouse, rat, goat, and rabbit) of one or more complementarity-determining regions (CDRs) that specifically bind to a desired antigen in a receptor human immunoglobulin molecule, and one or more human amino acid residues, which are amino acid residues that flank to CDRs, in the Fv framework region (FR). Such antibodies generally have reduced immunogenicity, and therefore have a longer half-life in humans compared to a non-human parent antibody from which one or more CDRs were obtained or on which they are based.

The term "chimera" and its grammatical variations as used herein in reference to an antibody, means that the amino acid sequence of the antibody contains one or more portions which are derived from, obtained or isolated from, or based on two or more different species. For example, a portion of the antibody may be human (e.g., constant region), and other portions of the antibody may be non-human (e.g., mouse heavy or mouse light chain variable region). Accordingly, an example of the chimeric antibody is an antibody in which different portions of the antibody are derived from different species. The chimeric antibody, unlike the humanized antibody, may have sequences of different species in any region of the antibody.

An antibody having at least partial sequence identity to any of the heavy chain variable region sequence or light chain variable region sequence of the antibody identified herein as the IL-10 inhibitor can be used as an antibody having the same function. The % identity of such antibodies and their subsequences may be as little as 60%, or may be higher than 60% (e.g., 65%, 70%, 75%, 80%, 85%, 90%, 95%, 96%, 97%, 98%, or 99%). In one embodiment of the present invention, an antibody having an identity of 65%, 70%, 75%, 80%, 85%, 90%, 95%, 96%, 97%, 98%, 99%, or the like in portions excluding CDRs of any of the variable regions of the antibody identified herein as the IL-10 inhibitor, can be used as the IL-10 inhibitor.

The term "identity" and its grammatical variations as used herein mean that two or more entities to be referred to are the same. Therefore, when two antibody sequences such as heavy or light variable region sequences are identical, they have the same amino acid sequence. The identity can extend over a defined area (region or domain) of the sequence. The "area, region, or domain" of homology or identity means that two or more entities to be referred to have homology, or are the same.

The extent of identity between two sequences can be confirmed using computer programs and mathematical algorithms known in the art. Such algorithms that calculate sequence identity (homology) generally account for sequence gaps and mismatches over the regions or areas to be compared. For example, BLAST (e.g., BLAST2.0) search algorithm (see, e.g., Altschul et al., J. Mol. Biol. 215:403 (1990), publicly available through NCBI) has typical search parameters as follows: Mismatch-2; gap open 5; gap extension 2. For polypeptide sequence comparison, BLASTP algorithm is generally used in combination with a scoring matrix such as PAM100, PAM 250, BLOSUM 62, or BLOSUM 50. FASTA (e.g., FASTA2 and FASTA3), and SSEARCH sequence comparison programs are also used to quantify the extent of identity (Pearson et al., Proc. Natl. Acad. Sci. USA 85:2444 (1988); Pearson, Methods Mol Biol. 132:185 (2000); and Smith et al., J. Mol. Biol. 147:195 (1981)). Programs to quantify protein structural similarity using Delaunay-based topological mapping have also been developed (Bostick et al., Biochem Biophys Res Commun. 304:320 (2003)).

The term "substantially identical" as used herein in reference to the binding affinity or avidity of an antibody or binding fragment for an antigen, means that the binding is within 100-fold (higher or lower) of the binding affinity of a reference antibody for the antigen (e.g., IL-10R). The binding affinity can be determined by rates of association (Kₐ) and dissociation (KD or K_{d}). The equilibrium affinity constant, K, is the ratio of Kₐ/K_{d}. The rates of association (Kₐ) and dissociation (KD or K_{d}) can be measured using surface plasmon resonance (SPR) (Rich and Myszka, Curr. Opin. Biotechnol. 11:54 (2000); Englebienne, Analyst. 123:1599 (1998)). Apparatuses and methods for real-time detecting and monitoring of binding rates are known and commercially available (BiaCore 2000, Biacore AB, Upsala, Sweden; and Malmqvist, Biochem. Soc. Trans. 27:335 (1999)). Therefore, for example, if the binding of a reference antibody to IL-10R or IFNAR has a KD of 10⁻⁹ M, an antibody having substantially identical binding affinity to the reference antibody will have a KD within the range from 10⁻⁷ M to KD 10⁻¹¹ M for the binding to IL-10R or IFNAR.

The term "substantially identical" as used in reference to amino acid sequences, means that the sequence difference between two amino acid sequences being compared is relatively small and the sequence difference has no substantial effects on specific binding properties to the antigen. A substantially identical amino acid sequence may include a partial modification of the amino acid sequence, for example, the amino acid sequence can be modified by deletion or substitution of 1 to several (e.g., 1 to 3) amino acids, or addition or insertion of 1 to several (e.g., 1 to 3) amino acids that constitute the amino acid sequence, or a combination thereof. The position of mutation in the amino acid sequence is not particularly limited, and mutations may occur at multiple positions. The number of amino acids modified in the amino acid sequence corresponds to, for example, within 10% of the total amino acids shown, and preferably corresponds to within 5% of the total amino acids. More preferably, the number corresponds to within 1% of the total amino acids.

The antibodies and binding fragments thereof described herein can have binding affinity, KD, for binding to IL-10R or IFNAR within about KD 10⁻² M to about KD 10⁻¹⁵ M, or within about KD 10⁻⁶ M to about KD 10⁻¹² M. In a particular embodiment, the binding affinity, KD, for binding to IL-10R is less than 5 × 10⁻² M, 10⁻² M, 5 × 10⁻³ M, 10⁻³ M, 5 × 10⁻⁴ M, 10⁻⁴ M, 5 × 10⁻⁵ M, 10⁻⁵ M, 5 × 10⁻⁶ M, 10⁻⁶ M, 5 × 10⁻⁷ M, 10⁻⁷ M, 5 × 10⁻⁸ M, 10⁻⁸ M, 5 × 10⁻⁹ M, 10⁻⁹ M, 5 × 10⁻¹⁰ M, 10⁻¹⁰ M, 5 × 10⁻¹¹ M, 10⁻¹¹ M, 5 × 10⁻¹² M, 10⁻¹² M, 5 × 10⁻¹³ M, 10⁻¹³ M, 5 × 10⁻¹⁴ M, 10⁻¹⁴ M, 5 × 10⁻¹⁵ M, and 10⁻¹⁵ M.

In one aspect of the present invention, modified forms or variants of the antibodies identified herein can be used as the IL-10 inhibitor. The term "modification" or "variant" as used herein means an antibody that retains at least partial activity or functions of the antibody before including modification or mutation.

Examples of modifications include one or more amino acid substitutions (e.g., 1 to 3, 3 to 5, 5 to 10, 10 to 15, 15 to 20, 20 to 25, or more residues), additions (e.g., insertions), and deletions (e.g., subsequences or fragments) in the constant or variable region sequences of the antibody. In a particular embodiment, an antibody of a modified form or a variant retains at least partial functions or activity of an unmodified antibody, for example, binding affinity (e.g., KD or K_{d}), or binding specificity to cells expressing an antigen, activity such as antagonistic activity of IL-10R, or inhibitory activity of IL-10/IL-10R signaling pathway. Such modified forms and variants can have less than, the same, or greater, but at least a part of functions or activity of a reference antibody or subsequence thereof, for example, binding to IL-10R, to reduce, decrease inhibit, suppress, limit, prevent or abrogate activity or functions of IL-10 or IL-10R, or the IL-10/IL-10R signaling pathway.

Examples of substitutions include conservative and non-conservative amino acid substitutions. Substitutions may be inside or outside the constant regions, complementarity-determining regions (CDRs), or framework regions (FRs) of the antibody. In a particular embodiment, a heavy or light chain CDR (CDR1, CDR2, or CDR3) or FR has 1 to 8, 1 to 5, 1 to 3, or fewer (e.g., 1 or 2) amino acid substitutions. In an additional embodiment, substitutions within a variable region sequence are not inside CDR. In another embodiment, substitutions within a variable region sequence are not inside FR. Particular, and non-limiting examples of amino acid substitutions include conservative substitutions inside or outside the constant regions, complementarity-determining regions (CDRs), or framework regions (FRs), for example, substitutions of one or more amino acid residues of the constant region, or a heavy or light chain variable region sequence, or a heavy chain variable region sequence, or a light chain variable region sequence.

The term "conservative substitution" as used herein refers to a substitution of one amino acid by a biologically, chemically, or structurally similar residue. Biologically similar means that the substitution does not destroy biological activity. Structurally similar means that the amino acids have side chains of similar length or similar size, such as alanine, glycine, and serine. Chemical similarity means that the residues have the same charge, or both are hydrophilic or hydrophobic. Specific examples thereof include a substitution of one hydrophobic residue with another, such as isoleucine, valine, leucine, or methionine; or substitution of one polar residue with another, such as the substitution of arginine with lysine, the substitution of glutamic acid with aspartic acid, the substitution of glutamine with asparagine, or substitution of serine with threonine. Conservative substitutions are also known as conservative amino acid substitution to those skilled in the art, and include examples shown in the following table (e.g., WO2010/146550).

**[Table 1]**

| Original residue | Conservative substitution | Exemplary substitution |
|---|---|---|
| Ala (A) | Val | Val; Leu; Ile |
| Arg (R) | Lys | Lys; Gln; Asn |
| Asn (N) | Gln | Gln; His; Asp, Lys; Arg |
| Asp (D) | Glu | Glu; Asn |
| Cys (C) | Ser | Ser; Ala |
| Gln (Q) | Asn | Asn; Glu |
| Glu (E) | Asp | Asp; Gln |
| Gly (G) | Ala | Ala |
| His (H) | Arg | Asn; Gln; Lys; Arg |
| Ile (I) | Leu | Leu; Val; Met; Ala; Phe; Norleucine |
| Leu (L) | Ile | Norleucine ; Ile; Val; Met; Ala; Phe |
| Lys (K) | Arg | Arg; Gln; Asn |
| Met (M) | Leu | Leu; Phe; Ile |
| Phe (F) | Tyr | Leu; Val; Ile; Ala; Tyr |
| Pro (P) | Ala | Ala |
| Ser (S) | Thr | Thr |
| Thr (T) | Ser | Ser |
| Trp (W) | Tyr | Tyr; Phe |
| Tyr (Y) | Phe | Trp; Phe; Thr; Ser |
| Val (V) | Leu | Ile; Leu; Met; Phe; Ala; Norleucine |

In addition, naturally occurring amino acids are, based on common side chain properties, classified into the following groups
(1) Non-polar Norleucine, Met, Ala, Val, Leu, Ile,
(2) Polar, uncharged Cys, Ser, Thr, Asn, Gln,
(3) Acidic (negatively charged) Asp, Glu,
(4) Basic (positively charged) Lys, Arg, His,
(5) Residues that affect chain orientation Gly, Pro, and
(6) Aromatic: Trp, Tyr, Phe.

As one embodiment, amino acids that belong to the same group as the amino acid to be substituted may be used for substitution.

Structural determinants that contribute to antigen binding, such as complementarity-determining regions (CDRs) and framework regions (FRs) in hypervariable regions are known in the art. The location of additional regions such as the D- and J-regions are also known. Antibodies and subsequences thereof containing one or more CDR sequences, optionally with flanking FR sequences, generally have sufficient sequence identity to a heavy or light chain variable region sequence illustrated herein, in order to retain at least partial functions or activity of an antibody containing heavy and light chain sequences illustrated herein, for example, binding affinity (e.g., KD), avidity or binding specificity or selectivity to the antigen.

One or a few amino acid substitutions (e.g., 2, 3, 4, or 5) in heavy or light chain variable region inside or outside CDRs are highly likely to be tolerated. Non-conservative substitutions of a large number of amino acids in hypervariable regions are highly likely to affect binding activity, specificity or antibody functions or activity. Regional mutation analysis can be used for predicting effects of particular substitutions in the complementarity-determining regions (CDRs) and framework regions (FRs) (Shapiro et al., J Immunol. 163:259 (1999)). In short, sequence comparison indicates a mutational hierarchy among di- and tri-nucleotide sequences located within Ig intronic DNA, and thereby predicting regions more or less susceptible to mutations. Quantitative structure-activity relationship (QSAR) can be used to identify the nature of the antibody recognition domain, and therefore the amino acids involved in ligand binding. Predictive models based on OSAR can be then used to predict effects of substitutions (mutations). For example, mutational effects on the association and dissociation rates of the antibody that interacts with the antigen can be used to construct quantitative predictive models for both kinetic constants (Kₐ and K_{d}), which can in turn be used to predict the effects of other mutations on the antibody (De Genst et al., J Biol Chem. 277:29897 (2002)). Therefore, one skilled in the art can use such analysis to predict amino acid substitutions of antibodies and subsequences that are highly likely to result in an antibody or subsequence retaining at least partial activity or functions of the unsubstituted antibody or subsequence.

The addition can be a covalent or non-covalent binding of any type of molecule to the antibody. Specific examples of antibody addition include glycosylation, acetylation, phosphorylation, amidation, formylation, ubiquitination, and derivatization with protective/blocking groups, and any of numerous chemical modifications. An additional specific but non-limiting example of addition is another amino acid sequence. **In** a specific embodiment, the addition is an amino acid sequence having one or more molecules not normally present in a fusion (chimeric) sequence, reference native (wild-type) sequence, that are covalently attached to that sequence. A particular example thereof is an amino acid sequence of another antibody for producing an antibody multimer, such as a multispecific antibody.

Another particular example of modified antibodies having amino acid addition is one in which a second heterologous sequence, in other words, heterologous functional domain is attached (covalent or non-covalent binding) that imparts a distinct or complementary function to the antibody. The heterologous functional domain is not limited to amino acid residues. Therefore, the heterologous functional domain can be composed of any of various different types of small or large functional components. Such components include nucleic acids, peptides, carbohydrates, lipids or low molecular weight organic compounds, such as drugs (e.g., antiviral agents), metals (gold and silver), and radioisotopes. For example, a tag such as T7 or polyhistidine can be attached to an antibody to facilitate purification or detection of an antigen. Thus, in other embodiments, the present invention provides an antibody and a heterologous domain, and the domain imparts a distinct function, in other words, a heterologous functional domain to the antibody.

Linkers, such as amino acid or peptidimimetic sequences may be inserted between the antibody sequence and the addition (e.g., heterologous functional domain) so that the two entities at least partially maintain a distinct function or activity. Linkers may have one or more properties that include a plastic conformation, an inability to form an ordered secondary structure or a hydrophobicity or charged properties which could promote or interact with either domain. Amino acids generally found in plastic protein regions include Gly, Asn and Ser. Other nearly neutral amino acids such as Thr and Ala can also be used in the linker sequence. The length of the linker sequence may vary without significantly affecting functions or activity of the fusion protein (see, e.g., U.S. Patent No. 6,087,329). Linkers further include chemical moieties and conjugating agents, such as sulfo-succinimidyl derivatives (sulfo-SMCC, sulfo-SMPB), disuccinimidyl suberate (DSS), disuccinimidyl glutarate (DSG), and disuccinimidyl tartrate (DST).

An additional non-limiting example of addition is a detectable label. Accordingly, in another embodiment, the present invention provides an IL-10R antibody and subsequence thereof that are detectably labeled. Specific examples of detectable labels include fluorophores, chromophores, radioisotopes (e.g., S¹⁵, P³², and I¹²⁵), electron-dense reagents, enzymes, ligands, and receptors. Enzymes are generally detected by their activity. For example, horseradish peroxidase is usually detected by its ability to convert a substrate such as 3,3',5,5'-tetramethylbenzidine (TMB) to a blue pigment, which can be quantified. Ligands can bind to other molecules such as biotin, which can bind to avidin or streptavidin, and IgG, which can bind to protein A.

Another example of addition is the insertion of amino acids into antibodies. Insertions may occur inside or outside CDRs or FRs, or in constant or variable regions such as heavy or light chain variable region sequences. Insertions in CDRs such as CDR3 naturally occur during antibody affinity maturation.

Additional specific and non-limiting examples of modifications and variants include antibody subsequences and fragments. The terms "functional subsequence" and "binding fragment" when referring to an antibody, mean a portion that retains at least a part of one or more functions or activity as a full-length or native antibody, for example, functions or activity of the original antibody, such as binding to an antigen. In one aspect of the present invention, antibody subsequences or fragments that bind to IL-10R, or fragments of IL-10R are considered to be functional subsequences. Antibody subsequences or fragments retain at least a part of the functions or activity of an unmodified or a reference full-length, native, or intact antibody. Subsequences or fragments have less than, the same, or greater affinity or avidity compared to a full-length native antibody, less than, the same, or greater binding specificity compared to a full-length native antibody, or one or more activities or functions that are less than, the same, or greater, compared to a full-length native antibody, for example, a function or activity of an IL-10R antibody.

In one aspect of the present invention, antibody subsequences or fragments that bind to IFNAR, or fragments of IFNAR are considered to be functional subsequences. Antibody subsequences or fragments retain at least a part of the functions or activity of an unmodified or a reference full-length, native, or intact antibody. Subsequences or fragments have less than, the same, or greater affinity or avidity compared to a full-length native antibody, less than, the same, or greater binding specificity compared to a full-length native antibody, or one or more activities or functions that are less than, the same, or greater, compared to a full-length native antibody, for example, a function or activity of an IFNAR antibody.

Typical subsequences and fragments include antibody subsequences and fragments that bind to antigens, such as antibodies having at least one fewer amino acid than the full-length antibody (e.g., one or more internal or terminal amino acid deletions from either amino or carboxy terminus of the antibody having two heavy chains and two light chains that bind to the antigen). Antibody subsequences and fragments containing single-chain antibodies can include all or part of heavy or light chain variable region sequences alone or in combination with all or part of one or more of the following: a hinge region, CH1, CH2, and CH3 domains. Non-limiting representative examples of fragments and subsequences of a full-length antibody include, but are not limited to, Fab, Fab', F(ab')₂, Fv, Fd, single-chain Fv (scFv), disulfide-linked Fv (sdFv), V_{L}, V_{H}, trispecific (Fab₃), bispecific (Fab₂), diabody ((V_{L}-V_{H})₂ or (V_{H}-V_{L})₂), triabody (trivalent), tetrabody (tetravalent), minibody ((scFV-C_{H}3)₂), bispecific single-chain Fv (Bis-scFv), IgGdeltaCH2, scFv-Fc, (scFv)₂-Fc, and IgG4PE.

Antibody subsequences and fragments can be combined. For example, V_{L} or V_{H} subsequences can be linked by a linker sequence, thereby forming a V_{L}-V_{H} chimera. A combination of single-chain Fv (scFv) subsequences can be linked by a linker sequence, thereby forming a scFv-scFv chimera. Antibody subsequences and fragments contain single-chain antibodies or variable regions alone or in combination with all or part of other antibody subsequences.

Binding fragments and subsequences also include a heavy or light chain variable region, or all or part of a full-length antibody heavy or light chain, which includes one, two, or three CDRs of a heavy or light chain variable region sequence, optionally with or without a flanking FR. In various forms, the full-length antibody heavy or light chain, or the binding fragment or subsequence of the heavy or light chain variable region, has about 20 to 30, 30 to 50, 50 to 100, 100 to 150, 150 to 200, 200 to 250, 250 to 300, 300 to 400, or 400 to 500 amino acid residues in length.

Another particular and non-limiting example of modification is a case where an antibody is, for example, altered to have a different isotype or subclass due to the substitution of the heavy chain constant region. The alteration of Ig subclass can result in a change or improvement in functions or activity (e.g., anti-IL-10R activity). Modifications thus include deleting small and large regions of the amino acid sequences from an antibody, and substituting the deleted region with another amino acid sequence.

Modified polypeptides also include one or more D-amino acids substituted for L-amino acids (and mixtures thereof), structural and functional analogs, for example, peptidomimetics having synthetic or unnatural amino acids or amino acid analogs, and derivatized forms. Modifications include cyclic structures such as end-to-end amide bonds between the amino and carboxy termini of the molecule, or intra- or inter-molecular disulfide bonds. Polypeptides may be modified in vitro or in vivo, for example, post-translationally modified to include, for example, sugar residues, phosphate groups, ubiquitin, fatty acids, and lipids.

Methods for producing polyclonal and monoclonal antibodies are known in the art. For example, IL-10R or an immunogenic fragment thereof is optionally conjugated to a carrier such as keyhole-limpet hemocyanin (KLH) or ovalbumin (e.g., BSA), or mixed with an adjuvant such as Freund's complete or incomplete adjuvant, and used to immunize animals. Using hybridoma technology, splenocytes derived from immunized animals that respond to IL-10R can be isolated and fused with myeloma cells. Monoclonal antibodies produced by hybridomas can be screened for reactivity with IL-10R or an immunogenic fragment thereof. Hybridoma, recombinant, and phage display methods are known in the art (see, e.g., U.S. Patent Nos. 4,902,614, 4,543,439, and 4,411,993; see, also Monoclonal Antibodies, Hybridomas: A New Dimension in Biological Analyses, Plenum Press, Kennett, McKearn, and Bechtol (eds.), 1980; and Harlow et al., Antibodies: A Laboratory Manual, Cold Spring Harbor Laboratory Press, 2nd ed. 1988).

Animals that may be immunized include primates, mice, rats, rabbits, goats, sheep, cattle, or guinea pigs. Initial and optional subsequent immunization may be through intravenous, intraperitoneal, intramuscular, or subcutaneous routes. In addition to that, to increase the immune response, an antigen can be conjugated to another protein such as ovalbumin or keyhole limpet hemocyanin (KLH), thyroglobulin and tetanus toxoid, or mixed with an adjuvant such as Freund's complete or incomplete adjuvant. Initial and optional subsequent immunization may be through intraperitoneal, intramuscular, intraocular, or subcutaneous routes. Subsequent immunization may be at the same or different concentrations of the antigen, and may be at regular or irregular intervals.

Animals include mammals genetically modified to include human gene loci, such as human immunoglobulin lambda or kappa light chain, which can be used to produce human antibodies. Transgenic (e.g., transchromosomic) animals having one or more human immunoglobulin genes are described, for example, in U.S. Patent No. 5,939,598; and International Publication Nos. 02/43478 and 02/092812. Human transchromosomic mice (KM mice (trademark)) are described, for example, in International Publication Nos. 02/43478 and 02/092812; and Ishida, et al., IBC's 11th Antibody Engineering Meeting. Abstract (2000)). Such animals include, for example, mice, rats, guinea pigs, rabbits, sheep, cows, pigs, and horses.

Humanized antibodies can be produced using techniques known in the art including, for example, CDR grafting (European patent application publication No. 239,400; International Publication No. 91/09967; U.S. Patent Nos. 5,225,539, 5,530,101, and 5,585,089), veneering or resurfacing (EP 592,106; EP 519,596; Padlan, Molecular Immunol. 28:489 (1991); Studnicka et al., Protein Engineering 7:805 (1994); Roguska. et al., Proc. Nat'l Acad. Sci. USA 91:969 (1994)), and chain shuffling (U.S. Patent No. 5,565,332). Human consensus sequences (Padlan, Mol. Immunol. 31:169 (1994); and Padlan, Mol. Immunol. 28:489 (1991)) have previously used to produce humanized antibodies (Carter et al., Proc. Natl. Acad. Sci. USA 89:4285 (1992); and Presta et al., J. Immunol. 151:2623 (1993)). Additional methods for producing human polyclonal antibodies and human monoclonal antibodies are described (see, e.g., Kuroiwa et al., Nat. Biotechnol. 20:889 (2002); International Publication Nos. 98/24893, 92/01047; 96/34096; 96/33735; U.S. Patent Nos. 5,413,923; 5,625,126; 5,633,425; 5,569,825; 5,661,016; 5,545,806; 5,814,318; 5,885,793; 5,916,771; and 5,939,598).

Methods for producing chimeric antibodies are known in the art (e.g., Morrison, Science 229:1202 (1985); Oi et al., BioTechniques 4:214 (1986); Gillies et al., J. Immunol. Methods 125:191 (1989); and U.S. Patent Nos. 5,807,715; 4,816,567; and 4,816,397). Chimeric antibodies in which a variable domain derived from an antibody of one species is substituted with the variable domain of another species are described, for example, in Munro, Nature 312:597 (1984); Neuberger et al., Nature 312:604 (1984); Sharon et al., Nature 309:364 (1984); Morrison et al., Proc. Nat'l. Acad. Sci. USA 81:6851 (1984); Boulianne et al., Nature 312:643 (1984); Capon et al., Nature 337:525 (1989); and Traunecker et al., Nature 339:68 (1989).

IL-10R protein or IFNAR protein suitable for generating antibodies can be produced by any of a variety of standard protein purification or recombinant expression techniques. Forms of IL-10R or IFNAR suitable for generating an immune response include IL-10R subsequences, such as immunogenic fragments. Additional forms of IL-10R or IFNAR include IL-10R expressing cells, IL-10R or IFNAR containing preparations or cell extracts or fractions, and partially purified IL-10R or IFNAR. For example, an IL-10R or IFNAR sequence can be produced by standard peptide synthesis techniques, such as solid phase synthesis. A portion of the protein may contain an amino acid sequence such as a T7 tag or polyhistidine sequence to facilitate purification of expressed or synthesized protein. The protein can be expressed in a cell and purified. The protein may be expressed as a part of a larger protein (e.g., fusion or chimera) by a recombinant method.

Suitable techniques that additionally may be employed in antibody methods include IL-10R- or IFNAR-based affinity purification, non-denaturing gel purification, HPLC or RP-HPLC, size exclusion, purification on protein A column, or any combination of these techniques. An antibody isotype can be determined using an ELISA assay, and for example, a human Ig can be identified using mouse Ig-absorbed anti-human Ig.

Polypeptide sequences including modified forms can be produced using a recombinant DNA technology via cell expression or in vitro translation. Polypeptide sequences including modified forms can also be produced by chemical synthesis using methods known in the art, for example, an automated peptide synthesis apparatus (e.g., Applied Biosystems, Foster City, CA).

Antibody subsequences and fragments can be prepared by proteolytic hydrolysis of antibodies, for example, by pepsin or papain digestion of whole antibodies. Antibody subsequences and fragments produced by enzymatic cleavage with pepsin provide a 5S fragment denoted F(ab')₂. This fragment can be further cleaved using a thiol reducing agent to produce 3.5S Fab' monovalent fragments. Alternatively, an enzymatic cleavage using pepsin directly produces two monovalent Fab' fragments and the Fc fragment (see, e.g., U.S. Patent Nos. 4,036,945 and 4,331,647; and Edelman et al., Methods Enymol. 1:422 (1967)). Other methods of cleaving antibodies, such as separation of heavy chains to form monovalent light-heavy chain fragments, further cleavage of fragments, or other enzymatic or chemical methods may also be used.

The terms "nucleic acid" and "polynucleotide" and the like refer to at least two or more ribo- or deoxy-ribonucleic acid base pairs (nucleotides) that are linked through a phosphoester bond or equivalent. Nucleic acids include polynucleotides and polynucleosides. Nucleic acids include single-stranded, double-stranded, or triple-stranded, circular, or linear molecules. Typical nucleic acids include, but are not limited to, RNA, DNA, cDNA, genomic nucleic acid, and naturally occurring and non-naturally occurring nucleic acids such as synthetic nucleic acid.

Nucleic acids can have various lengths. The lengths of nucleic acids generally range from about 20 nucleotides to 20 Kb, or any numerical value or range within or encompassing such lengths, 10 nucleotides to 10 Kb, 1 to 5 Kb or less, 1000 to about 500 nucleotides or less in length.
Nucleotides may also be shorter, for example, 100 to about 500 nucleotides, or about 12 to 25, 25 to 50, 50 to 100, 100 to 250, or about 250 to 500 nucleotides in length, or any numerical value, range, or value within or encompassing such lengths. In a particular embodiment, the nucleic acid sequence has a length of about 10 to 20, 20 to 30, 30 to 50, 50 to 100, 100 to 150, 150 to 200, 200 to 250, 250 to 300, 300 to 400, 400 to 500, 500 to 1000, or 1000 to 2000 nucleotides, or any numerical value, range, or value within or encompassing such lengths. Shorter polynucleotides are commonly referred to as "oligonucleotides" or "probes" of single-stranded or double-stranded DNA. However, there is no upper limit to the length of such oligonucleotides.

The term "complementary" or "antisense" refers to a polynucleotide or peptide nucleic acid capable of binding to a specific DNA or RNA sequence. Antisense includes a single-, double-, triple-, or greater stranded RNA and DNA polynucleotides and peptide nucleic acids (PNA) that bind RNA transcript or DNA. Particular examples thereof include RNA and DNA antisense that bind to sense RNA. For example, a single-stranded nucleic acid can target a protein transcript involved in metabolism, catabolism, removal, or degradation of glycogen derived from a cell (e.g., mRNA). Antisense molecules are generally 95 to 100% complementary to the sense strand, but may be "partially" complementary, in which only some of the nucleotides bind to the sense molecules (less than 100% complementary, e.g., 95%, 90%, 80%, 70%, and sometimes less), or any numerical value or range within or encompassing such percent values.

The term "hybridize" and grammatical variations thereof refer to the binding between nucleic acid sequences. Hybridizing sequences are generally about 50% or more complementary to the nucleic acid that encodes the amino acid sequence of the reference antibody or subsequence (e.g., antibody heavy or light chain variable region sequence). The hybridization region between hybridizing sequences is generally at least about 12 to 15 nucleotides, 15 to 20 nucleotides, 20 to 30 nucleotides, 30 to 50 nucleotides, 50 to 100 nucleotides, 100 to 200 nucleotides or more, or any numerical value or range within or encompassing such lengths.

Nucleic acid sequences further include nucleotide and nucleoside substitutions, additions and deletions, as well as derivatized forms and fusion/chimeric sequences (e.g., those that encode recombinant polypeptide).

Nucleic acids can be produced using various standard cloning and chemical synthesis techniques. The techniques include, but are not limited to, nucleic acid amplification, for example, polymerase chain reaction, with genomic DNA or cDNA targets using a primer (e.g., degenerate primer mixture) capable of annealing to an antibody encoding sequence. Nucleic acids can also be produced by chemical synthesis (e.g., solid phase phosphoramidite synthesis) or transcription from a gene. The sequences produced can then be translated in vitro, or cloned into a plasmid and propagated, and then expressed in a cell (e.g., host cell such as yeast or bacterium in an animal or plant, and eukaryotic or mammalian cell).

The nucleic acid can be inserted into a nucleic acid construct in which expression of the nucleic acid is influenced or adjusted by an "expression control element". The "expression control element" refers to a nucleic acid sequence element that adjusts or influences the expression of a nucleic acid sequence to which it is operably linked. The expression control element includes, as necessary, a promoter, an enhancer, a transcription terminator, a gene silencer, a start codon (e.g., ATG) in front of a protein-encoding gene, and the like.

The expression control element operatively linked to a nucleic acid sequence controls transcription and, as necessary, translation of the nucleic acid sequence. The expression control element includes an element that structurally activates transcription, that is inducible (i.e., requires an external signal for activation), derepressible (i.e., requires a signal to turn transcription off; when the signal is no longer present, transcription is activated or "derepressed"), or specific to cell-types or tissues (i.e., tissue-specific control element).

The nucleic acid may be inserted into a plasmid for propagation into a host cell and for subsequent genetic engineering. The plasmid is a nucleic acid that can be propagated in a host cell, the plasmid may optionally contain expression control elements in order to drive expression of the nucleic acid that encodes IL-10R binding antibody, subsequence thereof or antigen (e.g., IL-10R alpha, IL-10R beta, IFNAR1, or IFNAR2) in the host cell. A vector is used herein as a synonym for plasmid and may also include an expression control element for expression in a host cell (e.g., expression vector). The plasmid and vector generally contain at least an origin of replication for propagation in a cell and a promoter. The plasmid and vector are therefore useful for genetic engineering and expression of IL-10R binding antibodies and subsequences, as well as antibody constant, heavy and light chain variable regions as well as antigen (e.g., IL-10R or IFNAR). Accordingly, the vector that includes nucleic acids that encode or are complementary to IL-10R binding antibodies, or IFNAR binding antibodies, and subsequences thereof, as well as antibody constant, heavy and light chain variable regions are provided.

The nucleic acids that encode variable regions of the antibody heavy and light chains or subsequences thereof, or that encode the full-length IL-10R antibody heavy and light chains or subsequences thereof, can be produced synthetically or using a recombinant method, or isolated from a cell such as a hybridoma. The isolated nucleic acids may be inserted into a suitable expression vector, and introduced into suitable host cells (e.g., CHO, plant, and other cells) which can be cultured for the production of recombinant antibodies, heavy and light chains or subsequences thereof of interest.

For the production of antibodies, host cells that express or are transformed with nucleic acids that encode antibodies and subsequences of interest are used. The host cells include, but are not limited to, prokaryotic and eukaryotic cells such as bacteria, fungi (yeast), plant, insect, and animal (e.g., mammalian including primate and human, CHO cells and hybridoma) cells. Examples thereof include bacteria transformed with recombinant bacteriophage nucleic acids, plasmid nucleic acid or cosmid nucleic acid expression vectors; yeast transformed with recombinant yeast expression vectors; plant cell lines infected with recombinant virus expression vectors (e.g., cauliflower mosaic virus, CaMV, tobacco mosaic virus, TMV) or transformed with recombinant plasmid expression vectors (e.g., Ti plasmid); insect cell lines infected with recombinant virus expression vectors (e.g., baculovirus); and animal cell lines infected with recombinant virus expression vectors (e.g., retroviruses, adenovirus, vaccinia virus), or transformed animal cell lines engineered for stable expression.

The cells may be ex vivo or in a subject (in vivo), such as a primary cell isolate, cell culture (e.g., passaged, established or immortalized cell line), or part of a plurality of cells, or a tissue or organ. In a particular embodiment, the host cells are CHO cells, hybridoma cells, or HEK293F cells.

The term "transformed" or "transfected" when used in reference to a cell (e.g., a host cell) or organism, means a genetic change in a cell following the incorporation of an exogenous molecule, for example, a protein or nucleic acid (e.g., a transgene) into the cell. The "transfected" or "transformed" cell is therefore a cell into which an exogenous molecule has artificially been introduced, for example, by a recombinant DNA technique, or a progeny thereof.

The nucleic acid or protein can be stably or transiently transfected or transformed (expressed) in the cell and progeny thereof. The cells can be propagated and the introduced protein can be expressed, or nucleic acid can be transcribed. A progeny of a transfected or transformed cell may not be identical to the parent cell, since there may be mutations that occur during replication.

Introduction of the protein and nucleic acid into target cells (e.g., host cells) can also be carried out by a method known in the art such as an osmotic shock method (e.g., calcium phosphate), electroporation, microinjection, cell fusion, and the like. Introduction of the nucleic acid and polypeptide in vitro, ex vivo, and in vivo can also be accomplished using other techniques. Examples thereof include polymeric substances, such as polyesters, polyamine acids, hydrogel, polyvinyl pyrrolidone, ethylene-vinylacetate, methylcellulose, carboxymethylcellulose, protamine sulfate, or lactide/glycolide copolymers, polylactide/glycolide copolymers, or ethylenevinylacetate copolymers. The nucleic acid can be entrapped in microcapsules prepared by a coacervation technique or interfacial polymerization, for example, by use of hydroxymethylcellulose or gelatin-microcapsules, or poly(methylmethacrolate) microcapsules, respectively, or in a colloid system. Colloidal dispersion systems include macromolecule complexes, nanocapsules, microspheres, beads, and lipid-based systems, including oil-in-water emulsions, micelles, mixed micelles, and liposomes.

Liposomes for introducing various compositions into cells are known in the art and include, for example, phosphatidylcholine, phosphatidylserine, lipofectin, and DOTAP (e.g., U.S. Patent Nos. 4,844,904, 5,000,959, 4,863,740, and 4,975,282; and GIBCO-BRL, Gaithersburg, Md.). Piperazine-based amphiphilic cationic lipids useful for gene therapy are also known (see, e.g., U.S. Patent No. 5,861,397). Cationic lipid systems are also known (see, e.g., U.S. Patent No. 5,459,127). Polymeric substances, microcapsules, and colloidal dispersion systems such as liposomes are collectively referred to herein as "vesicles". Therefore, delivery of viral and non-viral vector means into cells, tissue, or organs, in vitro, in vivo, and ex vivo is included.

In one aspect of the present invention, the effects of the present invention are achieved by decreasing, reducing, inhibiting, preventing, blocking, or suppressing IL-10/IL-10R signaling. In one aspect of the present invention, decreasing, inhibiting, reducing, preventing, suppressing, or blocking IL-10 or IL-10R signaling results in signaling in IL-10R expressed in alveolar macrophages.

In one aspect of the present invention, the pharmaceutical composition may be administered by any administration or delivery method, or by any route, systemic, topical and local administration or delivery. Examples of typical administration and delivery routes include intravenous (i.v.), intraperitoneal (i.p.), intraarterial, intramuscular, parenteral, subcutaneous, intrapleural, topical, dermal, intradermic, transdermal, transmucosal, intracranial, intraspinal, rectal, oral (gastrointestinal system), mucosal, inhalation, respiration, intranasal, intubation, intrapulmonary, intrapulmonary instillation, buccal, sublingual, intravascular, intrathecal, intraluminal, iontophoretic, intraocular, ophthalmic, optical, intraglandular, intraorgan, and intralymphatic administration.

In one aspect of the present invention, the pharmaceutical composition may be administered in combination with other pharmaceuticals. In one embodiment, the administration may be performed simultaneously with, sequentially with, or separately from administration of other pharmaceuticals, and may be performed in single or multiple doses. In one aspect of the present invention, the pharmaceutical composition may be administered, for example, one or more times hourly, daily, weekly, monthly, or annually, or every about 1 to 10 weeks. The administration method is determined by a health care professional based on the SARS-CoV-2 infection situation, the lesions caused thereby, or the onset, progression, severity, frequency, and duration of one or more symptoms or complications associated with or caused by adverse symptoms, conditions, or complications, and the like. In one embodiment, the administration can be performed to an individual who is not infected with SARS-CoV-2.

In one aspect of the present invention, doses of the pharmaceutical composition can be determined based on current existing protocols using animal disease models, or optionally based on experiments on human clinical trials. Typical doses are in a range from about 0.1 mg/kg to about 100 mg/kg, and any numerical value, range, or value within such range. The dose can be adjusted according to the mass of a patient, and is generally 2, 3, 4 or more times hourly, weekly, monthly, or annually, and within a range from about 1 to 10 µg/kg, 10 to 25 µg/kg, 25 to 50 µg/kg, 50 to 100 µg/kg, 100 to 500 µg/kg, 500 to 1,000 µg/kg, 1 to 5 mg/kg, 5 to 10 mg/kg, 10 to 20 mg/kg, 20 to 50 mg/kg, 50 to 100 mg/kg, 100 to 250 mg/kg, 250 to 500 mg/kg, or greater. Typical ranges are from about 0.3 mg/kg to about 50 mg/kg, 0 to 25 mg/kg, or 1.0 to 10 mg/kg, or any numerical value, range, or value within such range.

In one aspect of the present invention, the doses of the pharmaceutical composition can vary, and depend on whether the treatment is preventive or therapeutic, the onset, progression, severity, frequency, duration probability or susceptibility of the symptoms, conditions, lesions or complications, the type of pathogen infection or pathogenic mechanism, the reactivation from latency or vaccination or immunization toward which treatment is directed, the desired clinical endpoint, previous or concurrent treatment, patient's general health, age, sex, race or immunocompetence, and other factors as appreciated by those skilled in the art. A person skilled in the art will recognize the factors that may influence the dosage and timing required to provide a sufficient amount for providing therapeutic or preventive benefits.

In one aspect of the present invention, the pharmaceutical composition is administered for therapeutic treatment as soon as possible within 1 to 2, 2 to 4, 4 to 12, 12 to 24, or 24 to 72 hours after a patient has been exposed or brought in contact with SARS-CoV-2, or within 1 to 2, 2 to 4, 4 to 12, 12 to 24, or 24 to 48 hours after the SARS-CoV-2 infection or the onset from latency, or the onset or development of one or more adverse symptoms, conditions, lesions, complications, or the like caused thereby. In one aspect of the present invention, the pharmaceutical composition can be administered for the durations of 0 to 4 weeks, for example, 2 to 3 weeks, for preventive treatment within 1 to 2, 2 to 4, 4 to 12, 12 to 24, 24 to 48, or 48 to 72 hours before the exposure to, contact with, or infection with SARS-CoV-2 or a pathogen.

As used herein the term "pharmaceutically acceptable" means one that can be used in pharmaceuticals. In one aspect of the present invention, the pharmaceutical composition is formulated a biologically acceptable formulation, gas, liquid, or solid, or a mixture thereof, suitable for one or more routes of administration, in vivo delivery or contact. Such formulations include solvents (aqueous or nonaqueous), solutions (aqueous or nonaqueous), emulsions (e.g., oil-in-water or water-in-oil), suspensions, syrups, elixirs, dispersion and suspension media, coatings, isotonic and absorption promoting or delaying agents, compatible with pharmaceutical administration, or in vivo contact or delivery. Aqueous and nonaqueous solvents, solutions, and suspensions can include suspending agents and thickening agents. Such pharmaceutically acceptable carriers include tablets (coated or uncoated), capsules (hard or soft), microbeads, powder, granules and crystals. Supplementary active compounds (e.g., preservatives, antibacterial agents, antiviral agents, and antifungal agents) can also be incorporated into the composition. The above additives are not particularly limited as long as they are pharmaceutically acceptable additives.

In one aspect of the present invention, the pharmaceutical composition can be formed to be compatible with particular administration routes. Accordingly, the pharmaceutical composition includes carriers, diluents, or excipients suitable for administration by various routes. Typical routes of administration for contact or in vivo delivery in which a composition can optionally be formulated include inhalation, respiration, intranasal, intubation, intrapulmonary instillation, oral, buccal, intrapulmonary, intradermal, topical, dermal, parenteral, sublingual, subcutaneous, intravascular, intrathecal, intraarticular, intraluminal, transdermal, iontophoretic, intraocular, ophthalmic, optical, intravenous (i.v.), intramuscular, intraglandular, intraorgan, and intralymphatic.

In one aspect of the present invention, the formulations suitable for parenteral administration include aqueous and non-aqueous solutions, suspensions, or emulsions of the active compound, which formulations are generally sterile and can be isotonic with the blood of an intended recipient. Non-limiting and illustrative examples include water, physiological saline, dextrose, fructose, ethanol, animal, plant, or synthetic oils.

For transmucosal or transdermal administration (e.g., topical contact), penetrants can be included in the pharmaceutical composition. Penetrants are known in the art, and include, for example, for transmucosal administration, detergents, bile salts, and fusidic acid derivatives. For transdermal administration, the active ingredient can be formed into aerosols, sprays, ointments, salves, gels, or creams as generally known in the art. For contact with skin, the pharmaceutical compositions generally include ointments, creams, lotions, pastes, gels, sprays, aerosols, or oils. Carriers which may be used include vaseline, lanolin, polyethylene glycols, alcohols, transdermal promoting agents, and combinations thereof.

Cosolvents and adjuvants may be added to the formulations. Non-limiting examples of cosolvents contain hydroxyl groups or other polar groups, for example, alcohols, such as isopropyl alcohol; glycols, such as propylene glycol, polyethyleneglycol, polypropylene glycol, and glycol ether; glycerol; polyoxyethylene alcohols and polyoxyethylene fatty acid esters. Adjuvants include, for example, surfactants such as soya lecithin and oleic acid; sorbitan esters such as sorbitan trioleate; and polyvinylpyrrolidone.

Supplementary compounds (e.g., preservatives, antioxidants, antimicrobial agents including biocides and biostats such as antibacterial, antiviral and antifungal agents) can also be incorporated into the composition. The pharmaceutical composition may therefore include preservatives, antioxidants, and antimicrobial agents.

Preservatives can be used to inhibit microbial growth or increase stability of ingredients, thereby prolonging the shelf life of the pharmaceutical formulations. Suitable preservatives are known in the art and include, for example, EDTA, EGTA, benzalkonium chloride, or benzoic acid or benzoates such as sodium benzoate. Antioxidants include, for example, ascorbic acid, vitamin A, vitamin E, tocopherols, and similar vitamins or provitamins.

Pharmaceutical formulations and delivery systems suitable for the compositions and methods of the invention are known in the art (see, e.g., Remington: The Science and Practice of Pharmacy (2003) 20th ed., Mack Publishing Co., Easton, PA; Remington's Pharmaceutical Sciences (1990) 18th ed., Mack Publishing Co., Easton, PA; The Merck Index (1996) 12th ed., Merck Publishing Group, Whitehouse, N.J.; Pharmaceutical Principles of Solid Dosage Forms (1993), Technonic Publishing Co., Inc., Lancaster, Pa.; Ansel and Stoklosa, Pharmaceutical Calculations (2001) 11th ed., Lippincott Williams & Wilkins, Baltimore, MD; and Poznansky et al., Drug Delivery Systems (1980), R. L. Juliano, ed., Oxford, N.Y., pp. 253-315).

In one aspect of the present invention, the pharmaceutical composition, together with any adjunct, compound, composition, can be packaged in unit dosage form (capsules, tablets, troches, cachets, lozenges) for ease of administration and uniformity of dosage. The "unit dosage form" as used herein refers to physically discrete units suitable as a single dosage for a patient to be treated, and each unit, when administered in one or more doses, contains a predetermined amount of active ingredient optionally in association with a pharmaceutical carrier (excipient, diluent, vehicle, or filler) which is calculated to produce a desired effect (e.g., preventive or therapeutic effect). The unit dosage forms also include, for example, ampules and vials, which may include a composition in a lyophilized state or lyophilized state under reduced pressure, and a sterile liquid carrier, for example, can be added prior to administration or in vivo delivery. The unit dosage forms further include, for example, ampules and vials with liquid compositions disposed therein. Individual unit dosage forms can be included in multi-dose kits or containers. The pharmaceutical formulations can be packaged in a single or multiple unit dosage form for ease of administration and uniformity of dosage.

One aspect of the present invention provides kits containing an IL-10R antibody and subsequences thereof, optionally with a pathogen antigen, live or attenuated pathogen, a combination composition, and a pharmaceutical formulation thereof, packaged into a suitable packaging material. The kit generally contains a label or package insert including a description of the components or instructions for use in vitro, in vivo, or ex vivo, of the components therein. The kit can contain, for example, an IL-10 inhibitor alone (in a separate container or pack) or in combination (e.g., a mixture), or can contain other compounds, agents, drugs, or compositions.

The term "packaging material" as used herein refers to a physical structure housing the components of the kit. The packaging material can maintain the components in a sterilized state, and can be made of materials commonly used for such purposes (e.g., paper, corrugated fiber, glass, plastic, foil, ampules, vials, and tubes).

In one aspect of the present invention, the kit can contain a label or a package insert. The label or package insert includes a "printed matter", for example, is paper or cardboard, or affixed separately or to a component, a kit or packing material (e.g., box), or attached to an ampule, tube or vial containing a kit component. The label or insert can further include a computer readable medium such as a disk (e.g., floppy diskette, hard disk, flash memory), optical disk such as CD- or DVD-ROM/RAM, DVD, MP3, magnetic tape, or an electrical storage media such as RAM and ROM, or hybrids thereof such as magnetic/optical storage media, flash media or memory-type cards.

The label or insert can include identification information and mechanism of action of one or more components therein, dosage of the active ingredients including pharmacokinetics and pharmacodynamics, and clinical pharmacological action. The label or insert can include information identifying manufacturer information, lot numbers, manufacturer location and date.

The label or insert can include information on conditions, disorders or diseases (e.g., viral infection, vaccination or immunization) for which kit components may be used. The label or insert can include instructions for the clinician or patient for using one or more of the kit components in a method, treatment protocol, or therapeutic regimen. Instructions can include dosage amounts, frequency or duration, and instructions for performing any of the methods, treatment protocols, or preventive or therapeutic regimes described herein. Typical instructions include instructions for treating a pathogen infection or lesion, and instructions for providing a subject with protection against pathogen infection, lesion, or reactivation from latency.

The label or insert can include information on any benefit that a component may provide, such as preventive or therapeutic benefits. The label or insert can include information on potential adverse side effects, complications or reactions, such as warnings to the subject or clinician regarding situations where it would not be appropriate to use a particular composition. Adverse side effects or complications could also occur when the subject has taken, will be taking, or is currently taking one or more other drugs that may be incompatible with the composition, or the subject has undergone, will be undergoing, or is currently undergoing another treatment protocol or therapeutic regimen which would be incompatible with the composition, and, therefore, the instructions could include information regarding such contraindication.

In one aspect of the present invention, provided is a method for determining risk of exacerbation of the novel coronavirus (SARS-CoV-2) disease 2019 (COVID-19), and the method includes measuring the blood concentration of IL-10 in a subject, and determining that the subject has a high risk of the exacerbation when the obtained blood concentration of IL-10 is equal to or more than 8 pg/mL. Here, the blood concentration of IL-10 can be measured by a well-known method. In one embodiment, the blood concentration can be stimulated using a blood sample taken from a subject. Examples of the blood concentration of IL-10, which serves as a reference for determining the risk of the exacerbation, include 8 pg/mL, 9 pg/mL, 10 pg/mL, and 11 pg/mL.

Subjects to be determined by the above determination method is not particularly limited as long as they are mammals, capable of being infected with SARS-CoV-2. Examples thereof include elderly people at the age of 60 or older, at the age of 70 or older, at the age of 80 or older, or at the age of 90 or older, individuals with a pre-existing lung disease, pregnant women, individuals who have not been vaccinated against SARS-CoV-2, individuals infected with SARS-CoV-2, and individuals who are not infected with SARS-CoV-2.

In one aspect of the present invention, provided is a method for determining risk of exacerbation of the novel coronavirus (SARS-CoV-2) disease 2019 (COVID-19), and the method includes measuring the expression level of the IL-10 receptor in the alveolar macrophages of a subject, and determining that the subject has a high risk of the exacerbation when the obtained expression level of the IL-10 receptor is equal to or more than a predetermined level. Here, the expression level of IL-10 receptor can be measured by a well-known method. In one embodiment, the expression level can be measured using a specimen of lung tissue taken from a subject. Examples of the expression level of the IL-10 receptor, which serves as a reference for determining the risk of the exacerbation, include an expression level exceeding the average of measured values of samples taken from a plurality of subjects, an expression level exceeding the average by 10%, an expression level exceeding the average by 20%, an expression level exceeding the average by 30%, and an expression level exceeding the average by 40%.

Subjects to be determined by the above determination method is not particularly limited as long as they are mammals, capable of being infected with SARS-CoV-2. Examples thereof include elderly people at the age of 60 or older, at the age of 70 or older, at the age of 80 or older, or at the age of 90 or older, individuals with a pre-existing lung disease, pregnant women, individuals who have not been vaccinated against SARS-CoV-2, individuals infected with SARS-CoV-2, and individuals who are not infected with SARS-CoV-2.

In one aspect of the present invention, provided is a method for determining risk of exacerbation of the novel coronavirus (SARS-CoV-2) disease 2019 (COVID-19), and the method includes confirming the presence or absence of the CIDRE expression in a subject, and determining that the subject has a high risk of the exacerbation when the subject is confirmed with the CIDRE expression. Here, the presence or absence of the CIDRE expression can be confirmed by a well-known method. In one embodiment, the expression can be measured using a specimen of blood taken from a subject.

Subjects to be determined by the above determination method is not particularly limited as long as they are mammals, capable of being infected with SARS-CoV-2. Examples thereof include elderly people at the age of 60 or older, at the age of 70 or older, at the age of 80 or older, or at the age of 90 or older, individuals with a pre-existing lung disease, pregnant women, individuals who have not been vaccinated against SARS-CoV-2, individuals infected with SARS-CoV-2, and individuals who are not infected with SARS-CoV-2.

In one embodiment of the present invention, a specimen selected from alveolar macrophages, blood, urine, sputum, feces, and spinal fluid can be used as the specimen for performing the determination method.

In one aspect of the present invention, provided is a protein having all or part of the amino acid sequence of interferon-α receptor 2 and all or part of the amino acid sequence of IL-10 receptor β. In one aspect of the present invention, the protein has the binding activity to type I interferon and the binding activity to IL-10 when expressed in cells. In addition, in one aspect of the present invention, the protein has the binding activity to type I interferon and the binding activity to IL-10 when expressed in cells, and contributes to signaling through binding of IL-10. Further, in one aspect of the present invention, the protein, when expressed in cells, contributes to increased ACE2 expression in the cells by stimulation of IL-10. Furthermore, in one aspect of the present invention, the protein is involved in SARS-CoV-2 infection in cells such as alveolar macrophages. As used herein, the protein may be referred to as Covid-19 infectivity enhancing dual receptor (CIDRE or CiDRE).

In one aspect of the present invention, CIDRE is a fusion protein having all or part of the amino acid sequence of interferon-α receptor 2 and all or part of the amino acid sequence of IL-10 receptor β. In one embodiment of the present invention, CIDRE is a protein derived from a chimeric transcript of an IL10RB gene and an IFNAR2 gene. In one embodiment of the present invention, CIDRE is a protein derived from a chimeric transcript of a IL10RB gene and an IFNAR2 gene that is expressed in a subject with a mutation in the genomic region that controls alternative splicing of the IL10RB gene and/or the IFNAR2 gene. In one embodiment of the present invention, CIDRE is a protein derived from a chimeric transcript of a IL10RB gene and an IFNAR2 gene that is expressed in a subject with a mutation in the genomic region that controls alternative splicing of the IFNAR2 gene.

In one embodiment of the present invention, CIDRE has the amino acid sequence of SEQ ID NO: 1 or SEQ ID NO: 2, or has the amino acid sequence substantially identical thereto. In one aspect of the present invention, a substantially identical amino acid sequences may include a partial modification of the amino acid sequence, and for example, the amino acid sequence can be modified by deletion or substitution of 1 to several (e.g., 1 to 3) amino acids, or addition or insertion of 1 to several (e.g., 1 to 3) amino acids that constitute the amino acid sequence, or a combination thereof. The position of mutation in the amino acid sequence is not particularly limited, and mutations may occur at multiple positions. The number of amino acids modified in the amino acid sequence corresponds to, for example, within 10% of the total amino acids shown, and preferably corresponds to within 5% of the total amino acids. More preferably, the number corresponds to within 1% of the total amino acids.

In one aspect of the present invention, provided is a nucleic acid that encodes CIDRE. In one embodiment, the nucleic acid is RNA of SEQ ID NO: 3 or SEQ ID NO: 4, or DNA corresponding thereto.

In one aspect of the present invention, prior to the administration of the pharmaceutical composition, the expression levels of CIDRE may be measured in the subjects, subjects having the expression levels equal to or more than a predetermined expression level may be selected, and the pharmaceutical composition may be administered to the selected subjects. In one embodiment, the predetermined expression level is greater than the average expression level of a plurality of subjects, 1.2-fold, 1.5-fold, 1.8-fold, 2-fold, 3-fold, 4-fold, 5-fold, 6-fold, 7-fold, 8-fold, 9-fold, 10-fold, 11-fold, 12-fold, or 13-fold the average expression level. In one embodiment, CIDRE expression level may be measured by quantifying the amount of expressed protein, or, for example, by a technique such as measuring the amount of mRNA. In one embodiment, CIDRE expression level may be measured by measuring the expression level in a predetermined cell, for example, by measuring the expression level in immune cells, specifically in macrophages. In one embodiment, subjects in whom expression levels are to be measured are individuals who are not infected with SARS-CoV-2, individuals who have not developed COVID-19, or individuals who have developed COVID-19 with mild or moderate symptoms.

In one aspect of the present invention, prior to the administration of the pharmaceutical composition, the IL-10 blood concentrations may be measured in the subjects, subjects having the concentrations equal to or more than a predetermined value may be selected, and the pharmaceutical composition may be administered to the selected subjects. In one embodiment, the predetermined value is, for example, exceeding the average value of a plurality of subjects, specifically, 10%, 20%, 30%, 40%, 50%, 60%, 70%, 80%, 90%, 100%, 110%, 120%, 130%, 140%, or 150% of the average value. In another embodiment, the IL-10 blood concentration is, for example, 8 pg/mL, 9 pg/mL, 10 pg/mL, 11 pg/mL, 12 pg/mL, 13 pg/mL, 14 pg/mL, or 15 pg/mL. In one embodiment, the measurement of the IL-10 blood concentration may be replaced by a technique such as the measurement of the amount of mRNA, for example. In one embodiment, subjects in whom the concentrations are to be measured are individuals who are not infected with SARS-CoV-2, individuals who have not developed COVID-19, or individuals who have developed COVID-19 with mild or moderate symptoms.

In one aspect of the present invention, prior to the administration of the pharmaceutical composition, the expression levels of the IL-10 receptor may be measured in the subjects, subjects having the expression levels equal to or more than a predetermined value may be selected, and the pharmaceutical composition may be administered to the selected subjects. In one embodiment, the predetermined expression level is greater than the average expression level of a plurality of subjects, 1.2-fold, 1.5-fold, 1.8-fold, 2-fold, 3-fold, 4-fold, 5-fold, 6-fold, 7-fold, 8-fold, 9-fold, 10-fold, 11-fold, 12-fold, or 13-fold the average expression level. In one embodiment, the expression level of the IL-10 receptor may be measured by quantifying the amount of expressed protein, or, for example, by a technique such as measuring the amount of mRNA. In one embodiment, the expression level of the IL-10 receptor may be measured by measuring the expression level in a predetermined cell, for example, by measuring the expression level in immune cells, specifically in macrophages. In one embodiment, subjects in whom expression levels are to be measured are individuals who are not infected with SARS-CoV-2, individuals who have not developed COVID-19, or individuals who have developed COVID-19 with mild or moderate symptoms.

As used herein, the term "IL-10 receptor" is not particularly limited, as long as it is a protein having the binding activity to IL-10 as a ligand, and having a signaling function after binding to the ligand. Accordingly, the IL-10 receptor as used herein includes wild-type receptors or mutants thereof, including an IL-10 receptor α and an IL-10 receptor β, as well as CIDRE and mutants thereof, and encompasses receptors capable of binding to IL-10 and signaling after binding to the ligand.

In one aspect of the present invention, prior to the administration of the pharmaceutical composition, the expression levels of the chimeric transcript of the IL10RB gene and the IFNAR2 gene may be measured in the subjects, subjects having the expression levels equal to or more than a predetermined expression level may be selected, and the pharmaceutical composition may be administered to the selected subjects. In one embodiment, the predetermined expression level is greater than the average expression level of a plurality of subjects, 1.2-fold, 1.5-fold, 1.8-fold, 2-fold, 3-fold, 4-fold, 5-fold, 6-fold, 7-fold, 8-fold, 9-fold, 10-fold, 11-fold, 12-fold, or 13-fold the average expression level. In one embodiment, the expression level of the chimeric transcript may be measured by quantifying the amount of expressed protein, or, for example, by a technique such as measuring the amount of mRNA. In one embodiment, the chimeric transcript is mRNA that encodes CIDRE. In one embodiment, the expression level of the chimeric transcript may be measured by measuring the expression level in a predetermined cell, for example, by measuring the expression level in immune cells, specifically in macrophages. In one embodiment, subjects in whom expression levels are to be measured are individuals who are not infected with SARS-CoV-2, individuals who have not developed COVID-19, or individuals who have developed COVID-19 with mild or moderate symptoms.

In one aspect of the present invention, prior to the administration of the pharmaceutical composition, the presence or absence of a mutation in a genomic region that controls alternative splicing of the IL10RB gene and/or the IFNAR2 gene is confirmed in the subject, subjects with the mutation may be selected, and the pharmaceutical composition may be administered to the selected subjects. In one aspect of the present invention, prior to the administration of the pharmaceutical composition, the presence or absence of a mutation in a genomic region that controls alternative splicing of the IL10RB gene is confirmed in the subject, subjects with the mutation may be selected, and the pharmaceutical composition may be administered to the selected subjects. In one embodiment of the present invention, the mutation causes an increased expression level of the chimeric transcript of the IL10RB gene and the IFNAR2 gene, compared to the wild type. In one embodiment of the present invention, the mutation causes an increased expression level of mRNA that encodes CIDRE compared to the wild type. In one embodiment of the present invention, the mutation is a T/T mutation or a C/T mutation of rs13050728, more specifically, a T/T mutation (wild type: C/C mutation). In one embodiment, subjects whose mutation is confirmed are individuals who are not infected with SARS-CoV-2, individuals who have not developed COVID-19, or individuals who have developed COVID-19 with mild or moderate symptoms.

In the selection of subjects to be administered herein, when the average expression level and the average value of a plurality of subjects are set to be a reference, the average value is an amount or value calculated from the measurement results of a predetermined number of subjects. In one embodiment of the present invention, the predetermined number of subjects is, for example, 5 or more, 10 or more, 15 or more, 20 or more, 30 or more, 40 or more, 50 or more, or 100 or more.

In one aspect of the present invention, provided is a method for determining risk of exacerbation of the novel coronavirus (SARS-CoV-2) disease 2019 (COVID-19), and the method includes measuring the expression levels of CIDRE in subjects, selecting subjects having the expression levels equal to or more than a predetermined expression level, and determining that the selected subjects have a high risk of the exacerbation. In one embodiment, the predetermined expression level is greater than the average expression level of a plurality of subjects, 1.2-fold, 1.5-fold, 1.8-fold, 2-fold, 3-fold, 4-fold, 5-fold, 6-fold, 7-fold, 8-fold, 9-fold, 10-fold, 11-fold, 12-fold, or 13-fold the average expression level. In one embodiment, CIDRE expression level may be measured by quantifying the amount of expressed protein, or, for example, by a technique such as measuring the amount of mRNA. In one embodiment, CIDRE expression level may be measured by measuring the expression level in a predetermined cell, for example, by measuring the expression level in immune cells, specifically in macrophages. In one embodiment, subjects in whom expression levels are to be measured are individuals who are not infected with SARS-CoV-2, individuals who have not developed COVID-19, or individuals who have developed COVID-19 with mild or moderate symptoms.

In one aspect of the present invention, provided is a method for determining risk of exacerbation of the novel coronavirus (SARS-CoV-2) disease 2019 (COVID-19), and the method includes measuring the blood concentrations of IL-10 in subjects, selecting subjects having the concentrations equal to or more than a predetermined value, and determining that the selected subjects have a high risk of the exacerbation. In one embodiment, the predetermined value is, for example, exceeding the average value of a plurality of subjects, specifically, 10%, 20%, 30%, 40%, 50%, 60%, 70%, 80%, 90%, 100%, 110%, 120%, 130%, 140%, or 150% of the average value. In another embodiment, the IL-10 blood concentration is, for example, 8 pg/mL, 9 pg/mL, 10 pg/mL, 11 pg/mL, 12 pg/mL, 13 pg/mL, 14 pg/mL, or 15 pg/mL. In one embodiment, the measurement of the IL-10 blood concentration may be replaced by a technique such as the measurement of the amount of mRNA, for example. In one embodiment, subjects in whom the concentrations are to be measured are individuals who are not infected with SARS-CoV-2, individuals who have not developed COVID-19, or individuals who have developed COVID-19 with mild or moderate symptoms.

In one aspect of the present invention, provided is a method for determining risk of exacerbation of the novel coronavirus (SARS-CoV-2) disease 2019 (COVID-19), and the method includes measuring the expression levels of the IL-10 receptor in subjects, selecting subjects having the expression levels equal to or more than a predetermined expression level, and determining that the selected subjects have a high risk of the exacerbation. In one embodiment, the predetermined expression level is greater than the average expression level of a plurality of subjects, 1.2-fold, 1.5-fold, 1.8-fold, 2-fold, 3-fold, 4-fold, 5-fold, 6-fold, 7-fold, 8-fold, 9-fold, 10-fold, 11-fold, 12-fold, or 13-fold the average expression level. In one embodiment, the expression level of the IL-10 receptor may be measured by quantifying the amount of expressed protein, or, for example, by a technique such as measuring the amount of mRNA. In one embodiment, the expression level of the IL-10 receptor may be measured by measuring the expression level in a predetermined cell, for example, by measuring the expression level in immune cells, specifically in macrophages. In one embodiment, subjects in whom expression levels are to be measured are individuals who are not infected with SARS-CoV-2, individuals who have not developed COVID-19, or individuals who have developed COVID-19 with mild or moderate symptoms.

In one aspect of the present invention, provided is a method for determining risk of exacerbation of the novel coronavirus (SARS-CoV-2) disease 2019 (COVID-19), and the method includes measuring the expression levels of the chimeric transcript of the IL10RB gene and the IFNAR2 gene in subjects, selecting subjects having the expression levels equal to or more than a predetermined expression level, and determining that the selected subjects have a high risk of the exacerbation. In one embodiment, the predetermined expression level is greater than the average expression level of a plurality of subjects, 1.2-fold, 1.5-fold, 1.8-fold, 2-fold, 3-fold, 4-fold, 5-fold, 6-fold, 7-fold, 8-fold, 9-fold, 10-fold, 11-fold, 12-fold, or 13-fold the average expression level. In one embodiment, the expression level of the chimeric transcript may be measured by quantifying the amount of expressed protein, or, for example, by a technique such as measuring the amount of mRNA. In one embodiment, the chimeric transcript is mRNA that encodes CIDRE. In one embodiment, the expression level of the chimeric transcript may be measured by measuring the expression level in a predetermined cell, for example, by measuring the expression level in immune cells, specifically in macrophages. In one embodiment, subjects in whom expression levels are to be measured are individuals who are not infected with SARS-CoV-2, individuals who have not developed COVID-19, or individuals who have developed COVID-19 with mild or moderate symptoms.

Unless otherwise defined, all technical and scientific terms used herein have the same meaning as commonly understood by one of ordinary skill in the art to which this invention belongs. Although methods and materials similar or equivalent to those described herein can be used in the practice or testing of the present invention, suitable methods and materials are described herein.

### EXAMPLES

### [Test Animals]

C57BL/J mice, BALB/c mice, and golden hamsters were purchased from Japan SLC, Inc. Male mice of 6 to 8 week-old and male hamsters of 3 to 4 week-old were used for the experiments. All the animals were housed in individually ventilated cages with free access to food and water, in a room where the temperature and light were adjusted (23°C, light condition from 8 to 20 o'clock and dark condition from 20 to 8 o'clock) of a specific pathogen-free (SPF) facility.

### [Test Example 1] In Vivo Infection Experiment and RNA-seq

All animal experiments using SARS-CoV-2 were conducted in a biosafety level 3 (ABSL3) facility. The golden hamsters were anesthetized by intraperitoneal administration with 0.75 mg/kg medetomidine (Meiji Seika Pharma Co., Ltd.), 2 mg/kg midazolam (Sandoz AG), and 2.5 mg/kg butorphanol tartrate (Meiji Seika Pharma Co., Ltd.), and infected with 1.0 × 10⁶ PFU of SARS-CoV-2 (β strain) through the intranasal route.

SARS-CoV-2 (α strain, β strain, γ strain, and Omicron strain) used in the experiments was obtained from the National Institute of Infectious Diseases and cultured using VeroE6/TMPRSS2 cells cultured in DMEM containing 10% FCS and 100 U/ml penicillin-streptomycin.

RNA was collected from the lungs uninfected (after 0 days) and 5 days after infection, and a single-end cDNA library (TruSeq Stranded mRNA Library Prep (Illumina, Inc.)) was created using 200 ng of total RNA. The library was sequenced using Novaseq 6000 with 100-base single reads to be about 120 M reads for all samples.

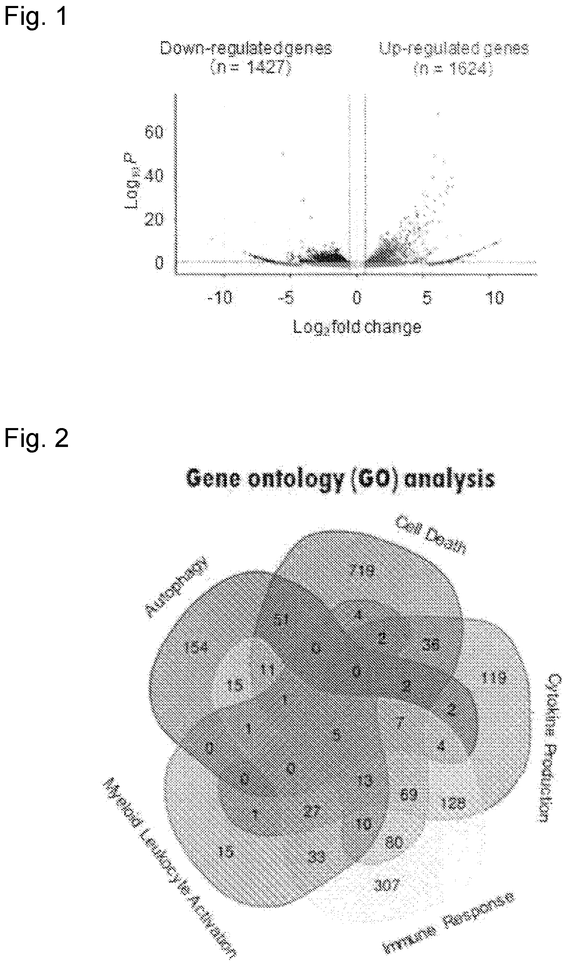

HISAT2 was used to perform alignments to the MesAur 1.0 golden hamster genome. Transcripts were assembled using StringTie (version 1.3.6). Gene counts and transcript counts were generated using StringTiesprepDE.py script. Raw count data from duplicates was analyzed using DEseq2 to obtain differentially expressed genes (DEGs) between Day 0 and Day 5. The volcano plot (Fig. 1) using the "EnhancedVolcano" (registered trademark) package indicated the significance of DEGs with p values less than 0.1. The heatmap was generated using the "heatmap.2" (registered trademark) package. It was confirmed that 1624 DEGs were upregulated.

A total of 5009 genes with Log₂FC exceeding 2 in Fig. 1 were selected and analyzed using g:Profiler (Nucleic Acids Research, 2019, Vol. 47, Web Server issue W191-W198). Since the histological analysis showed that cell death and autophagy predominantly occurred in COVID-19-infected lungs, "cell death", "autophagy", "immune response", "cytokine production", and "myeloid leukocyte activation" were selected from gene ontology (GO), and a Venn plot of significantly associated cells was created (Fig. 2).

As a result, 5 genes (TICAM1, IL10, TREM2, PSEN1, and PYCARD) that are predicted to play an important role in COVID-19 exacerbation were extracted, confirming that IL-10 was upregulated by SARS-CoV-2.

### [Test Example 2] Analysis of Blood Sample from Patients with Mild SARS-CoV-2 Infection

The concentrations of humoral factors were measured in blood samples (n=120) obtained from patients with SARS-CoV-2 infection who were at the early stage after the infection and whose severity was mild.

Human serum samples for ELISA were taken from 128 patients with COVID-19 in the bio-bank of Tokyo Medical and Dental University as serum samples collected post-hospital admission. All subjects signed a consent form approved by the Tokyo Medical and Dental University Ethical Committee. The ethical approval for human research was obtained from the Tokyo Medical and Dental University Ethical Committee. IL-10 (BioLegend, Inc., 430604), IL-6 (BioLegend, 430501), MCP-1 (BioLegend, 438804), IL-4 (BioLegend, 430301), GM-CSF (BioLegend, 432001), TNFα (BioLegend, 430201), IL-1β (BioLegend, 437004), IL-5 (BioLegend, 430401), IL-1α (BioLegend, 445804), IL-2 (BioLegend, 431801), IL-13 (BioLegend, 435207), IL-33 (BioLegend, 435907), IFNα (BioLegend, 446404), IFNγ (BioLegend, Inc., 430104), and IFNλ (R&D Systems, Inc., DY1598B-05) in human serum were measured by ELISA (BioLegend, Inc., or R&D Systems, Inc.) according to the manufacturers' protocols.

These results were analyzed for differences between patients with exacerbation (n=27) and patients without exacerbation (n=93) after sample collection. Note that patients who did not require admission to the ICU or use of a ventilator at the time of admission were determined to be subjects for blood sample collection, and those who were admitted to the ICU or use of a ventilator after obtaining the sample were determined to be patients with exacerbation, and other patients were determined to be patients without exacerbation (Novel Coronavirus Disease 2019 (COVID-19) Medical Treatment Guide, version 5.2, Ministry of Health, Labour and Welfare).

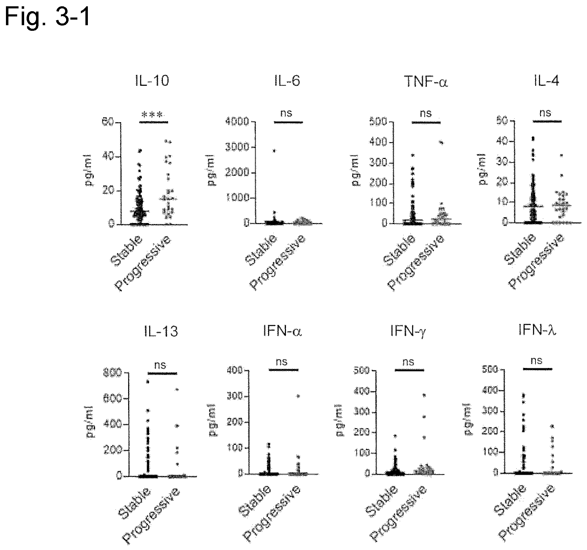

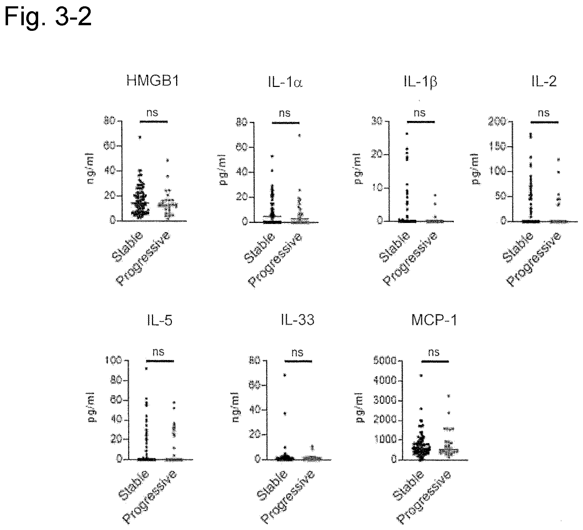

As a result, the IL-10 blood concentration of the patients with exacerbation was confirmed to be significantly higher than that of the patients without exacerbation (Fig. 3, p<0.005). The average of the IL-10 blood concentration of the patients with exacerbation was 15.20 pg/ml, whereas that of the patients without exacerbation was 7.483 pg/ml. On the other hand, no significant differences were confirmed for other humoral factors. When significant differences were confirmed by graphs herein, * indicates <0.05, ** indicates <0.01, *** indicates <0.005, and **** indicates <0.001.

### [Test EXAMPLE 3] In Vitro Infection Experiment Using Alveolar Macrophages

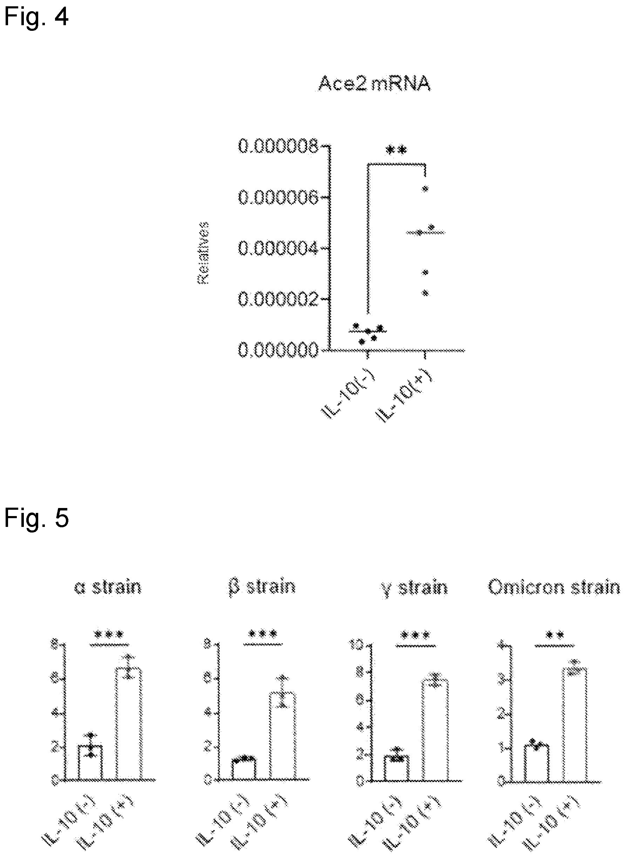

Bronchoalveolar lavage (BAL) cells obtained by intubating mice and washing the lungs with PBS were cultured at 37°C, 5% CO₂, in a complete RPMI medium for 4 hours, and then replaced with a fresh medium in the presence or absence of 40 ng/ml IL-10 (BioLegend, Inc., recombinant mouse IL-10 (carrier-free), Cat# 575806). After 24-hour cell culture, alveolar macrophages were infected with SARS-CoV-2 (β strain) at a multiplicity of infection (MOI) of 10. Cells were collected 72 hours after viral infection, and RNA was extracted using a High Pure RNA Isolation kit (Roche Diagnostics Corporation) or TRIzol (Thermo Fisher Scientific, Inc.). This RNA was reverse transcribed with ReverTra Ace qPCR RT Master Mix (TOYOBO Co., Ltd.), and then quantitative PCR (qPCR) was performed using THUNDERBIRD SYBR qPCR Mix (TOYOBO Co., Ltd.) on a LightCycler 480 system (Roche Diagnostics Corporation). The measurement results of ACE2 mRNA expression level are shown in Fig. 4.

Cells infected with α strain, β strain, γ strain, and Omicron strain were produced in the same manner, and the ACE2 mRNA expression levels were measured in the same manner. The results are shown in Fig. 5.

### [Test Example 4] Confirmation of ACE2 Expression by FACS

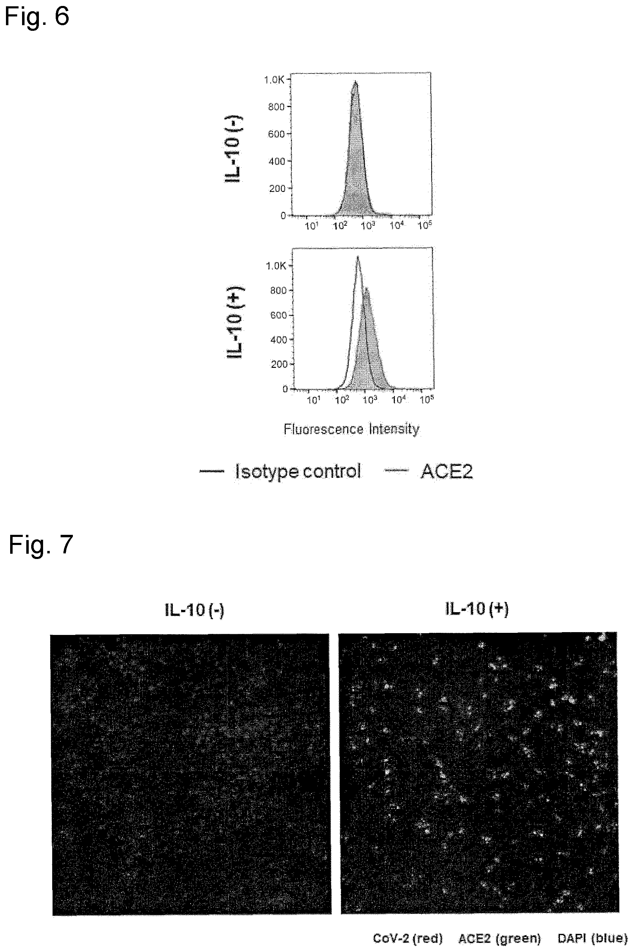

Using flow cytometry (FACS) analysis, isolated BAL samples (>95% alveolar macrophages) were incubated together with antibodies (see below) in FACS buffer (PBS containing 0.5% bovine serum albumin (BSA) and 2 mM EDTA; pH 7.2) at 4°C for 20 minutes. The alveolar macrophages were CD45⁺CD31⁻ CD11c⁺ SiglecF⁺ cells, and confirmed using CD45 antibody (BioLegend, Inc.; cat# 30-F11), anti-CD31 antibody (BioLegend, Inc.; clone# MEC13.3), antiCD11c antibody (BioLegend, Inc.; clone# N418), and anti-SiglecF antibody (BioLegend, Inc.; clone# S17007L). As in Test Example 3, culturing was performed at 37°C, 5% CO₂, in a complete RPMI medium for 4 hours, and then replaced with a fresh medium in the presence or absence of 40 ng/ml IL-10 (BioLegend, Inc., recombinant mouse IL-10 (carrier-free), Cat# 575806). After 24-hour cell culture, to evaluate the ACE2 expression, isolated BAL samples (>95% alveolar macrophages) were stained with anti-goat ACE2 antibody (1 µg/ml, AF933, R&D Systems, Inc.) at 4°C for 30 minutes, and washed with FACS buffer twice. Next, the cells were incubated together with Alexa Fluor 647 labeled anti-goat IgG antibody (ab150135, Abcam) at 4°C for 20 minutes. Data was obtained with a flow cytometer (FACS CantoII, BD Bioscience) and analyzed using FlowJo software (Tree Star, Inc.). In addition, the cells were selected and collected using FACS Aria III (BD Bioscience). The results are shown in Fig. 6. As a control, an isotype antibody (BioLegend, Inc., Ultra-LEAF (trademark) purified rat IgG1, κ Isotype Ctrl antibody, Clone: RTK207) was used.

### [Test Example 5] Confirmation of ACE Expression by Immunostaining

For the virus-infected cells of Test Example 3, the ACE expression was confirmed by the following immunostaining method.

Paraffin-embedded sections were deparaffinized using alcohol with gradually decreasing concentrations and washed with PBS. Antigen retrieval was performed using a Target Retrieval Solution (Dako, Inc.) in a Pascal pressure heating chamber (Dako, Inc.). Endogenous peroxidase was blocked using a peroxidase blocking solution (Dako, Inc.). Tissue was incubated with anti-ACE2 (1:100) or mouse anti-SARS-CoV-2 (1:1000) antibodies in antibody diluent (Dako, Inc.). Tissue was also stained with rabbit anti-LC3B (1:1000) antibody. EnVision + System-HRP labeled polymer and DAB (Dako, Inc.) were used as chromogens. Counterstaining was performed with hematoxylin. The results are shown in Fig. 7.

### [Test Example 6] Neutralization Experiment Using IL-10 Receptor Antibody (In Vitro, In Vivo)

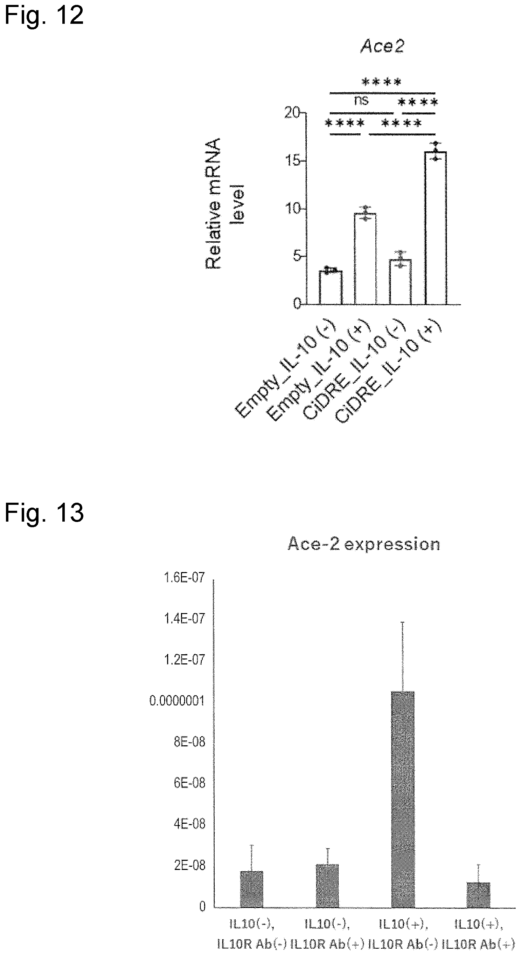

Collected BAL was cultured at 37°C, 5% CO₂, in a complete RPMI medium for 4 hours, and then the supernatant was replaced with a fresh medium in the presence or absence of 40 ng/ml IL-10, and 10 µg/ml anti-mouse IL-10 receptor inhibitory antibody (BioLegend, Inc.; Ultra-LEAF (trademark) purified anti-mouse CD210 (IL-10) antibody, Clone: 1B1.3a). After 24-hour incubation, alveolar macrophages were infected with SARS-CoV-2 (β strain) at MOI 10. Cells were collected 72 hours after viral infection and lysed for RNA extraction. Results of measuring the ACE2 expression level by quantitative PCR (qPCR) in the manner of Test Example 3 are shown in Fig. 13. It was confirmed that the ACE2 expression level increased with the addition of IL-10, whereas the expression level decreased with the addition of an anti-IL10R antibody.

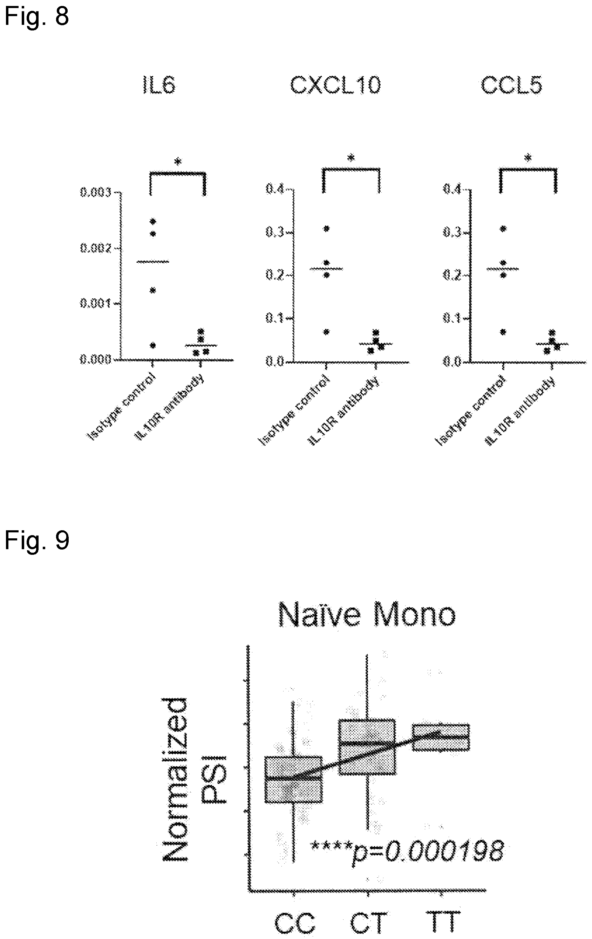

Golden hamsters were infected with coronavirus (β strain) using the method described above, and 2 days after infection, 400 µg/body of anti-IL-10 receptor B antibody (BioLegend, Inc.; Ultra-LEAF (trademark) purified anti-mouse CD210 (IL-10R) antibody, Clone: 1B1.3a) and an isotype control (BioLegend, Inc., Ultra-LEAF (trademark) purified rat IgG1, κ Isotype Ctrl antibody, Clone: RTK207) were administered daily, and collected and analyzed on Day 5. Quantitative PCR (qPCR) was performed using the method described in Test Example 3, and the relative values of gene expression of IL-6, CCL5, and IP 10 (CXCL10) were calculated using the standard curve method normalized to β-actin. The results are shown in Fig. 8.

### [Test Example 7] Analysis Of Genomic Regions (sQTL) that Control Alternative Splicing Regarding COVID-19 Disease Susceptibility SNPs at the IFNAR2-IL1-RB Gene Locus and Long-Read Capture RNA-seq

An xGenCustom target capture probe (biotinylated 120bp-ssDNA whose manufacturing is outsourced to Integrated DNA Technologies Inc. (IDT)) that covers the entire main isoform sequence of IFNAR2 and IL10RB and the junction sequence of the fusion transcript was prepared. TRIZOL reagent (Thermo Fisher Scientific, Inc.) after stimulation of THP-1 cells (ATCC, TIB-202 (trademark)) with phorbol myristate acetate (PMA, 10 ng/ml), lipopolysaccharide (LPS, 100 ng/ml) or IFNγ (10 ng/ml), and total RNA was collected and purified using the RNeasy Mini Kit (QIAGEN N.V.). Using Oligo dT primers, 100 ng total RNA was reverse transcribed according to the smartseqv2 protocol, and amplified with 22 cycles of PCR using KAPA HiFi Hot Start Ready Mix (Kapa Biosystems, Inc.) with 5Me-isodC-TSO and ISPCR primers. cDNA was hybridized and captured with xGen probes using the xGEN Hybridization and Wash Kit (IDT). The captured cDNA was then further amplified by 14 cycles of PCR as described above. The Nanopore Ligation Sequencing Kit (SQK-LSK109; Oxford Nanopore Technologies, plc) and NEBNext Quick Ligation Module/NEBNext Ultra II End-Repair/dA-Tailing Module (New England Biolabs, Inc.) were used for library preparation for sequencing. The cDNA was then sequenced on a Flongle Flow Cell (FLO-FLG001; Oxford Nanopore Technologies, plc). Base calling was done using Guppy (v4.4.1). The resulting fastq files were aligned to the GRCh38 primary assembly using minimap2v2.17 with reference to the splice junctions in the GENCODE38 annotation. The total length of the fusion isoform was identified using the flare pipeline and filtered using the following criteria. 1) isoforms that express more than 50 reads in total, 2) 5' end is FANTOM CAGE peak (TSS peak based on relaxed 0.14 threshold by TSS classifier), 3) 3' end is isoforms within 100 bp of TES of PolyASite2.0, 4) isoforms evaluated by CPATv3 to be protein-coding isoforms. 0.4 (coding probability ≥0.364).

The results of sQTL analysis are shown in Fig. 9. sQTL analysis confirmed a correlation of mutations (C/C, T/C, and T/T) at rs13050728 in the IL10RB genes with changes in splicing pattern. Expression of fusion transcripts of IFNAR2 and IL10RB, such as SEQ ID NOs: 1 and 3 was confirmed by long-read capture RNA-seq.

### [Test Example 8] Construction of CIDRE Expressing Macrophage Cell Line

As a primer, using Fw: GGATCC ATGCTTTTGAGCCAGAATGCC [BamHI] and Rv: CTCGAG CTAGCTTTGGGGCCCCTG [Xho1] (restriction enzyme sites are underlined), cDNA derived from a human macrophage line was used as a template for cloning. cDNA was prepared by a conventional method from RNA recovered using THP-1 (ATCC, TIB-202 (trademark)).

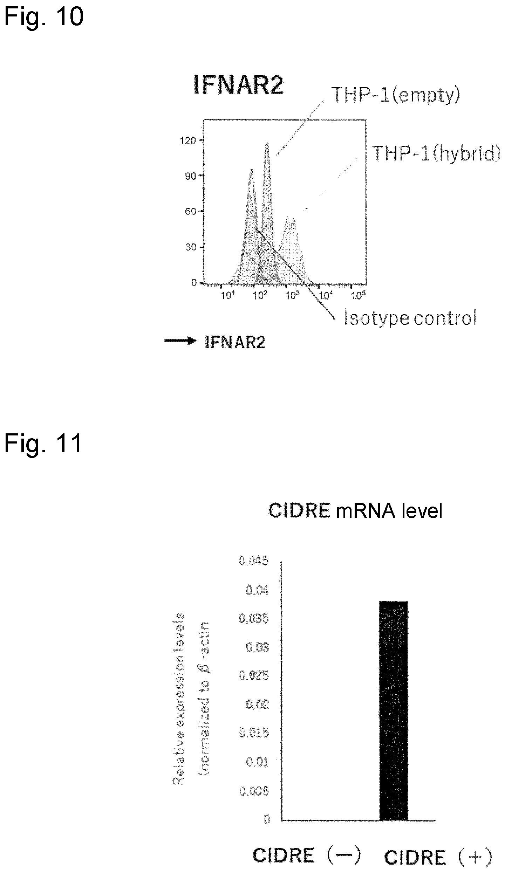

The obtained gene product was introduced into a lentivirus using the lentivirus system, and alveolar macrophages isolated from mice were infected with CiDRE-expressing lentivirus to cause expression. The results of confirming the expression by FACS are shown in Fig. 10. For FACS, anti-human IFNAR2 monoclonal antibody (Clone #122 (Sino Biological, Inc.), which recognizes a part of the hybrid sequence, Met1-Lys243) was used. From the results in Fig. 10, it was confirmed that the cell surface expression level of the IFNAR2 domain increased in the CIDRE-expressing strain, confirming that CIDRE was expressed on the cell surface.

Quantitative PCR (qPCR) was performed using the method described in Test Example 3, and the relative values of gene expression of CIDRE were calculated using the standard curve method normalized to β-actin. The results are shown in Fig. 11.

After one week, the cells in which the sufficient expression level was confirmed were stimulated with IL-10 (40 ng/ml, BioLegend, Inc.) for 48 hours using the same method as in Test Example 3, and then subjected to quantitative PCR (qPCR) using the method described above for ACE2 expression analysis. The results thereof are shown in Fig. 12.

### [Test Example 9] Measurement of CIDRE Expression Level by PCR

Quantitative PCR was performed with Roche LightCycler 480 using cDNA obtained from THP-1 (ATCC, TIB-202 (trademark)) as a template. The primers used are shown below.

### SYBR qPCR primer:

### CIDRE

Fw: aacatgagtggaaatttcaccta
Rv: ggtaccattcccaatgctgat
The PCR conditions are shown below.
pre-denature 60 sec
(45 cycles below)
denature 95°C 15 sec
annealing 60°C 15 sec
extension 72°C 45 sec

### [Test Example 10] Confirmation of CIDRE Signaling by IL-10 Stimulation

Human CIDRE (and an empty vector as a control) was expressed in mouse alveolar macrophages using a lentiviral vector.

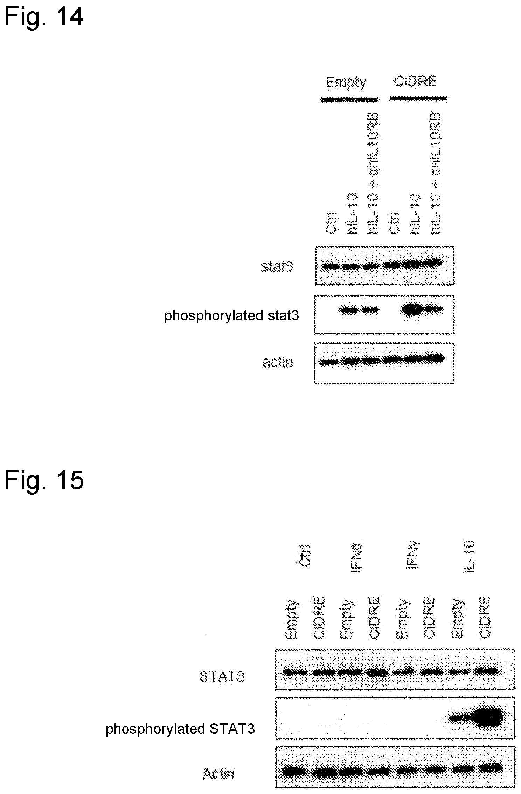

Lentiviruses were generated using HEK293T cells by cotransfection of the lentiviral expression plasmid FUIGW that encodes the human CIDRE transcript or FUIGW-Nluc (empty control) and two lentiviral helper plasmids. 48 hours after transfection, the culture supernatant containing lentivirus was collected, concentrated with Lenti-X (Clonetech), and passed through a 0.45 µm PES filter. Lentiviral infection of mouse alveolar macrophages was performed by culturing them for 7 days in the presence of 5 µg/cm² RetroNectin (Takara). Stable gene expression was confirmed by a GFP positive signal using BZ-X710 (KEYENCE CORPORATION). For functional analysis of CIDRE, cells successfully transduced were collected, human IL-10 inhibitory antibody (1 µg/ml (MAB874 (R&D Systems, Inc.)) was added to the culture medium, cultured for 30 minutes, and human IL-10 (40 ng /ml) was added and collected after incubation at 37°C for 10 minutes, and stat3, phosphorylated stat3, and Actin (CST) were confirmed by Western blotting. The results are shown in Fig. 14.

The human IL-10 is known to be capable of binding to mouse IL-10R and human IL-10R. On the other hand, the human IL-10 inhibitory antibody used this time exhibits an inhibitory effect by binding only to human IL-10R (the IL-10Rb portion of IL-10R), and does not bind to mouse IL-10R. It was confirmed that when Empty-expressing cells were stimulated with human IL-10, stat3 phosphorylation occurred, and that stat3 phosphorylation was not inhibited in the first place even when human IL-10Rb antibody was used (Fig. 14).

On the other hand, when mouse alveolar macrophages expressing human CIDRE were stimulated with human IL-10, the human IL-10 binds to mouse IL-10R and CIDRE, resulting in a further increase in phosphorylated stat3. It was confirmed that when this was inhibited using a human IL-10Rb antibody, only CIDRE was inhibited, and therefore, phosphorylation was suppressed accordingly (Fig. 14).

In addition, mouse alveolar macrophages successfully transduced using the above method were collected after a 10-minute incubation with IL-10 (40 ng/ml), IFNα (40 ng/ml), and IFNγ (40 ng/ml), and stat3, phosphorylated stat3 and Actin were confirmed by Western blotting. The results are shown in Fig. 15. Even when stimulated with IFNα, phosphorylation of Stat3 could not be confirmed in both the case of empty expression and the case of CIDRE expression. On the other hand, this was not observed due to stimulation with IL-10. Phosphorylation of Stat3 was confirmed in both the case of empty expression and the case of CIDRE expression, and an increase in the expression level of phosphorylated Stat3 was confirmed in CIDRE-expressing cells compared to empty-expressing cells (Fig. 15).

### [Test Example 11] Confirmation of Binding between CIDRE and IFNα

Human CIDRE (and empty vector as a control) was expressed in HEK293 using lipofectamin2000, and cultured using IFNα to confirm whether CIDRE and IFNα bind.

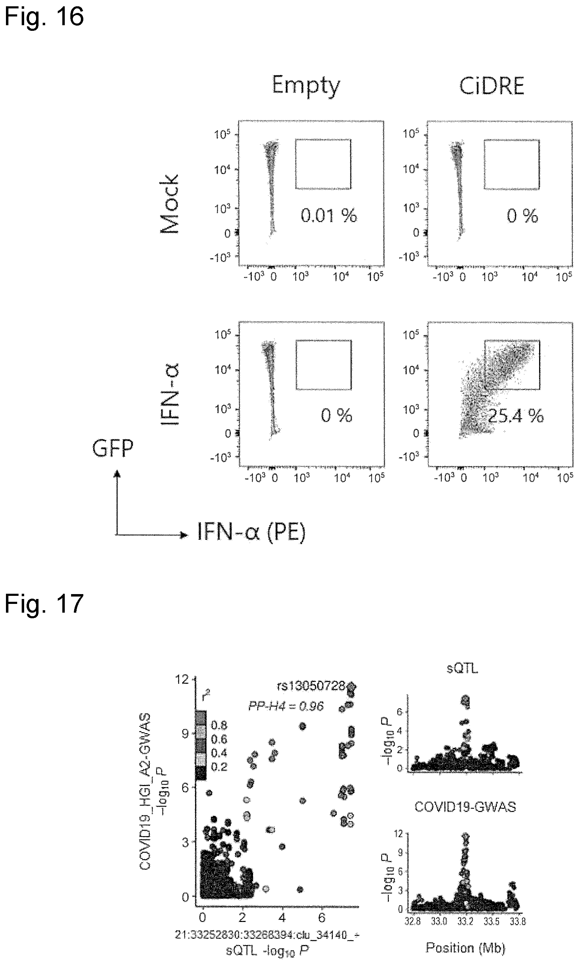

HEK293 cells were transfected with expression plasmid FUIGW that encodes human CIDRE transcript or FUIGW-Nluc (empty control) using lipofectamin2000, and 48 hours later, cultured at 4°C for 1 hour. Furthermore, IFNα was added and cultured at 4°C for 3 hours. After culturing, cells were collected, stained with PE-anti-IFNα antibody, and analyzed using FACS. The results are shown in Fig. 16. In the CIDRE-expressing cell group, where the fluorescence intensity of GFP derived from lentivirus and expressing is high, an increase in the fluorescence intensity derived from IFNα was observed, confirming that IFNα binds to the surface of cells expressing human CIDRE. Also, in combination with the results of Test Example 10, it was confirmed that even when INFα binds to CIDRE, there is no signaling.

### [Test Example 12] Colocalization Analysis of Signals Obtained by sQTL and GWAS

Regarding the COVID-19 exacerbation, a colocalization analysis was performed on the correlation between the signals obtained from sQTL and GWAS.

GWAS (COVID-19 HGI Release 5 (https://storage.googleapis.com/covid19-hg-public/20201215/results/20210107/COVID19_HGI_A2_ALL_eur_leave_23andme_2021010 7.b37.txt.gz)) related to the COVID-19 exacerbation was used. For the evaluation of colocalization of sQTL and GWAS signals, a Bayesian framework was applied using coloc R package63. A 500,000 bp window centered on the GWAS lead variant was tested and defined a PP-H4 (posterior probability of shared causal variant) > 0.8 as the dominant value. Plots were created using LocusCompare. The results thereof are shown in Fig. 17. It was confirmed that there is a highly significant correlation between the mutation (T/T) that causes the sQTL confirmed in Test Example 7 and the CIDRE expression.

### [Test Example 13] Confirmation of Influence of Presence or Absence of T/T Mutation on CIDRE Expression Level

Human PBMCs (purchased from Precision for Medicine, Inc.) were cultured with 20 ng/ml M-CSF (R&D Systems, Inc.) for 7 days and differentiated into macrophages. RNA was collected from differentiated macrophages using TRIZOL (Thermo Fisher Scientific, Inc.), total RNA was reverse transcribed using ReverTra Ace (TOYOBO Co., Ltd.) to synthesize cDNA, and this was used as a template for quantitative analysis using the following primers.
Fw;5'-AACATGAGTGGAAATTTCACCTA-3'
Rev; 5'-TGACAAGTTAATTCCAAACACGA-3'

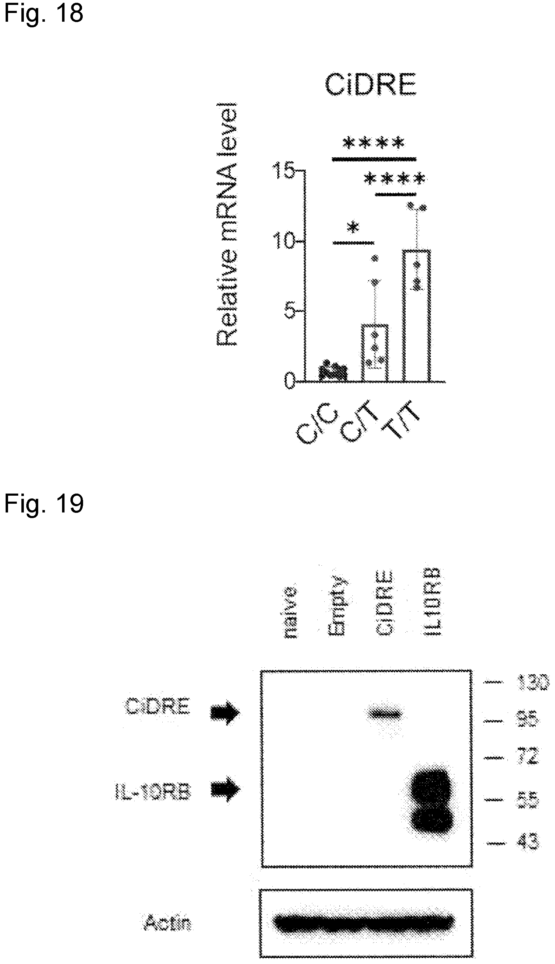

The presence or absence of the T/T mutation in the macrophages subjected to the above quantitative PCR was confirmed by the following method. For macrophages, the genomic DNA of KOD FX Neo (TOYOBO Co., Ltd.) and differentiated macrophages were combined using the following primer pair
Fw; 5'-gaggcatagtttcactctgttg-3',
Rev; 5'-ctggacacagtggctcatac-3'
for genotyping using a PCR reaction. The PCR product was subcloned into a plasmid using the sequencing primer 5'-cagtggctcatacctgtaacc-3'. The results are shown in Fig. 18. CIDRE was confirmed to be highly expressed in total RNA derived from cells with the T/T mutation. The number of people of each genotype is T/T: 5, C/T: 6, C/C: 11, and the average expression level ratio for each genotype is (T/T)/(C/C) = 13.10297, (T/C)/(C/C)=5.66472.

### [Test Example 14]

Using cells obtained by differentiating human PBMC into macrophages using the method described above, the size of CIDRE in comparison with IL-10Rb was confirmed by Western blotting.

The results are shown in Fig. 19. Normal IL-10Rb is detected on the right side of the right diagram, but in CIDRE, IFNar2 is bound to IL-10Rb, so the size detected using the same antibody is correspondingly larger (Fig. 19, left lane on the right diagram).

**[Table 2-1]**

| SEQ ID NO. | Sequence |
|---|---|
| 1 CIDRE transcr ipt (varian t 1) | ATGCTTTTGAGCCAGAATGCCTTCATCTTCAGATCACTTAATTTGGTTCTCA TGGTGTATATCAGCCTCGTGTTTGGTATTTCATATGATTCGCCTGATTACAC AGATGAATCTTGCACTTTCAAGATATCATTGCGAAATTTCCGGTCCATCTTA TCATGGGAATTAAAAAACCACTCCATTGTACCAACTCACTATACATTGCTGT ATACAATCATGAGTAAACCAGAAGATTTGAAGGTGGTTAAGAACTGTGCAAA TACCACAAGATCATTTTGTGACCTCACAGATGAGTGGAGAAGCACACACGAG GCCTATGTCACCGTCCTAGAAGGATTCAGCGGGAACACAACGTTGTTCAGTT GCTCACACAATTTCTGGCTGGCCATAGACATGTCTTTTGAACCACCAGAGTT TGAGATTGTTGGTTTTACCAACCACATTAATGTGATGGTGAAATTTCCATCT ATTGTTGAGGAAGAATTACAGTTTGATTTATCTCTCGTCATTGAAGAACAGT CAGAGGGAATTGTTAAGAAGCATAAACCCGAAATAAAAGGAAACATGAGTGG AAATTTCACCTATATCATTGACAAGTTAATTCCAAACACGAACTACTGTGTA TCTGTTTATTTAGAGCACAGTGATGAGCAAGCAGTAATAAAGTCTCCCTTAA AATGCACCCTCCTTCCACCTGGCCAGGAATCAGCATTGGGAATGGTACCAGC TCCCGAAAATGTCAGAATGAATTCTGTTAATTTCAAGAACATTCTACAGTGG GAGTCACCTGCTTTTGCCAAAGGGAACCTGACTTTCACAGCTCAGTACCTAA GTTATAGGATATTCCAAGATAAATGCATGAATACTACCTTGACGGAATGTGA TTTCTCAAGTCTTTCCAAGTATGGTGACCACACCTTGAGAGTCAGGGCTGAA TTTGCAGATGAGCATTCAGACTGGGTAAACATCACCTTCTGTCCTGTGGATG ACACCATTATTGGACCCCCTGGAATGCAAGTAGAAGTACTTGCTGATTCTTT ACATATGCGTTTCTTAGCCCCTAAAATTGAGAATGAATACGAAACTTGGACT ATGAAGAATGTGTATAACTCATGGACTTATAATGTGCAATACTGGAAAAACG GTACTGATGAAAAGTTTCAAATTACTCCCCAGTATGACTTTGAGGTCCTCAG AAACCTGGAGCCATGGACAACTTATTGTGTTCAAGTTCGAGGGTTTCTTCCT GATCGGAACAAAGCTGGGGAATGGAGTGAGCCTGTCTGTGAGCAAACAACCC ATGACGAAACGGTCCCCTCCTGGATGGTGGCCGTCATCCTCATGGCCTCGGT CTTCATGGTCTGCCTGGCACTCCTCGGCTGCTTCGCCTTGCTGTGGTGCGTT TACAAGAAGACAAAGTACGCCTTCTCCCCTAGGAATTCTCTTCCACAGCACC TGAAAGAGTTTTTGGGCCATCCTCATCATAACACACTTCTGTTTTTCTCCTT TCCATTGTCGGATGAGAATGATGTTTTTGACAAGCTAAGTGTCATTGCAGAA GACTCTGAGAGCGGCAAGCAGAATCCTGGTGACAGCTGCAGCCTCGGGACCC CGCCTGGGCAGGGGCCCCAAAGCTAG |
| Nucleotide sequence | |
| CIDRE (varian t 1) | MLLSQNAFIFRSLNLVLMVYISLVFGISYDSPDYTDESCTFKISLRNFRSIL SWELKNHSIVPTHYTLLYTIMSKPEDLKVVKNCANTTRSFCDLTDEWRSTHE AYVTVLEGFSGNTTLFSCSHNFWLAIDMSFEPPEFEIVGFTNHINVMVKFPS IVEEELQFDLSLVIEEQSEGIVKKHKPEIKGNMSGNFTYIIDKLIPNTNYCV SVYLEHSDEQAVIKSPLKCTLLPPGQESALGMVPPPENVRMNSVNFKNILQW ESPAFAKGNLTFTAQYLSYRIFQDKCMNTTLTECDFSSLSKYGDHTLRVRAE FADEHSDWVNITFCPVDDTIIGPPGMQVEVLADSLHMRFLAPKIENEYETWT MKNVYNSWTYNVQYWKNGTDEKFQITPQYDFEVLRNLEPWTTYCVQVRGFLP DRNKAGEWSEPVCEQTTHDETVPSWMVAVILMASVFMVCLALLGCFALLWCV YKKTKYAFSPRNSLPQHLKEFLGHPHHNTLLFFSFPLSDENDVFDKLSVIAE DSESGKQNPGDSCSLGTPPGQGPQS |
| Amino acid sequence | |

**[Table 2-2]**

| | |
|---|---|
| CIDRE transcr ipt (varian t 2) | ATGCTTTTGAGCCAGAATGCCTTCATCTTCAGATCACTTAATTTGGTTCTCA TGGTGTATATCAGCCTCGTGTTTGGTATTTCATATGATTCGCCTGATTACAC AGATGAATCTTGCACTTTCAAGATATCATTGCGAAATTTCCGGTCCATCTTA TCATGGGAATTAAAAAACCACTCCATTGTACCAACTCACTATACATTGCTGT ATACAATCATGAGTAAACCAGAAGATTTGAAGGTGGTTAAGAACTGTGCAAA TACCACAAGATCATTTTGTGACCTCACAGATGAGTGGAGAAGCACACACGAG GCCTATGTCACCGTCCTAGAAGGATTCAGCGGGAACACAACGTTGTTCAGTT GCTCACACAATTTCTGGCTGGCCATAGACATGTCTTTTGAACCACCAGAGTT TGAGATTGTTGGTTTTACCAACCACATTAATGTGATGGTGAAATTTCCATCT ATTGTTGAGGAAGAATTACAGTTTGATTTATCTCTCGTCATTGAAGAACAGT CAGAGGGAATTGTTAAGAAGCATAAACCCGAAATAAAAGGAAACATGAGTGG AAATTTCACCTATATCATTGACAAGTTAATTCCAAACACGAACTACTGTGTA TCTGTTTATTTAGAGCACAGTGATGAGCAAGCAGTAATAAAGTCTCCCTTAA AATGCACCCTCCTTCCACCTGGCCAGGAATCAGCATTGGGAATGGTACCACC TCCCGAAAATGTCAGAATGAATTCTGTTAATTTCAAGAACATTCTACAGTGG GAGTCACCTGCTTTTGCCGAAGGGAACCTGACTTTCACAGCTCAGTACCTAA GTTATAGGATATTCCAAGATAAATGCATGAATACTACCTTGACGGAATGTGA TTTCTCAAGTCTTTCCAAGTATGGTGACCACACCTTGAGAGTCAGGGCTGAA TTTGCAGATGAGCATTCAGACTGGGTAAACATCACCTTCTGTCCTGTGGATG ACACCATTATTGGACCCCCTGGAATGCAAGTAGAAGTACTTGCTGATTCTTT ACATATGCGTTTCTTAGCCCCTAAAATTGAGAATGAATACGAAACTTGGACT ATGAAGAATGTGTATAACTCATGGACTTATAATGTGCAATACTGGAAAAACG GTACTGATGAAAAGTTTCAAATTACTCCCCAGTATGACTTTGAGGTCCTCAG AAACCTGGAGCCATGGACAACTTATTGTGTTCAAGTTCGAGGGTTTCTTCCT GATCGGAACAAAGCTGGGGAATGGAGTGAGCCTGTCTGTGAGCAAACAACCC ATGACGAAACGGTCCCCTCCTGGATGGTGGCCGTCATCCTCATGGCCTCGGT CTTCATGGTCTGCCTGGCACTCCTCGGCTGCTTCGCCTTGCTGTGGTGCGTT TACAAGAAGACAAAGTACGCCTTCTCCCCTAGGAATTCTCTTCCACAGCACC TGAAAGAGTTTTTGGGCCATCCTCATCATAACACACTTCTGTTTTTCTCCTT TCCATTGTCGGATGAGAATGATGTTTTTGACAAGCTAAGTGTCATTGCAGAA GACTCTGAGAGCGGCAAGCAGAATCCTGGTGACAGCTGCAGCCTCGGGACCC CGCCTGGGCAGGGGCCCCAAAGCTAG |
| Nucleotide sequence | |
| CIDRE (varian t 2) | MLLSQNAFIFRSLNLVLMVYISLVFGISYDSPDYTDESCTFKISLRNFRSIL SWELKNHSIVPTHYTLLYTIMSKPEDLKVVKNCANTTRSFCDLTDEWRSTHE AYVTVLEGFSGNTTLFSCSHNFWLAIDMSFEPPEFEIVGFTNHINVMVKFPS IVEEELQFDLSLVIEEQSEGIVKKHKPEIKGNMSGNFTYIIDKLIPNTNYCV SVYLEHSDEQAVIKSPLKCTLLPPGQESALGMVPPPENVRMNSVNFKNILQW ESPAFAEGNLTFTAQYLSYRIFQDKCMNTTLTECDFSSLSKYGDHTLRVRAE FADEHSDWVNITFCPVDDTIIGPPGMQVEVLADSLHMRFLAPKIENEYETWT MKNVYNSWTYNVQYWKNGTDEKFQITPQYDFEVLRNLEPWTTYCVQVRGFLP DRNKAGEWSEPVCEQTTHDETVPSWMVAVILMASVFMVCLALLGCFALLWCV YKKTKYAFSPRNSLPQHLKEFLGHPHHNTLLFFSFPLSDENDVFDKLSVIAE DSESGKQNPGDSCSLGTPPGQGPQS |
| Amino acid sequence | |
| CIDRE Primer 1 (Fw) | GGATCCATGCTTTTGAGCCAGAATGCC |
| CIDRE Primer 1 (Rv) | CTCGAGCTAGCTTTGGGGCCCCTG |
| CIDRE Primer 2 for analysis | aacatgagtggaaatttcaccta |

**[Table 2-3]**

| | |
|---|---|
| (Fw) | |
| CIDRE Primer 2 for analysis (Rv) | ggtaccattcccaatgctgat |
| CIDRE Primer 3 for analysis (Rv) | tgacaagttaattccaaacacga |
| Primer for genotyping | gaggcatagtttcactctgttg |
| (Fw) | |
| Primer for genotyping | ctggacacagtggctcatac |
| (Rv) | |
| Primer for plasmid subcloning | cagtggctcatacctgtaacc |

## Claims

1. A pharmaceutical composition for use in therapy or prevention of viral infection in which ACE2 is used as a receptor, the composition containing an IL-10 inhibitor.

2. The pharmaceutical composition according to claim 1, wherein the viral infection is the novel coronavirus (SARS-CoV-2) disease 2019 (COVID-19).

3. The pharmaceutical composition according to claim 1 or 2, wherein the IL-10 inhibitor is an anti-IL-10 monoclonal antibody, an anti-IL-10 receptor monoclonal antibody, or an antigen-binding fragment thereof.

4. The pharmaceutical composition according to any one of claims 1 to 3, wherein the IL-10 inhibitor is an anti-IL-10 receptor β monoclonal antibody or an antigen-binding fragment thereof.

5. The pharmaceutical composition according to any one of claims 1 to 4, for use in therapy or prevention of exacerbation of COVID-19.

6. The pharmaceutical composition according to any one of claims 1 to 5, wherein the antibody or antigen-binding fragment is a full-length antibody, Fab, Fab', F(ab')₂, Fv, scFv, dsFv, a diabody, or sc(Fv)₂.

7. The pharmaceutical composition according to any one of claims 1 to 6, wherein the IL-10 inhibitor is a mouse antibody, a humanized antibody, a human antibody, a chimeric antibody, or an antigen-binding fragment thereof.

8. The pharmaceutical composition according to any one of claims 1 to 7, for use in therapy or prevention of exacerbation of COVID-19 accompanied by pneumonia.

9. The pharmaceutical composition according to any one of claims 1 to 8, for administration to a subject who has a measured blood concentration of IL-10 that is equal to or more than a predetermined value.

10. The pharmaceutical composition according to any one of claims 1 to 9, for administration to a subject who has been confirmed to have a measured expression level of a protein (CIDRE) that is equal to or more than a predetermined level, the protein having all or a part of the amino acid sequence of an interferon-α receptor 2 and all or a part of the amino acid sequence of an IL-10 receptor β.

11. The pharmaceutical composition according to any one of claims 1 to 10, for administration to a subject who has been determined to be expected to have a therapeutic or preventive effect of administration of the IL-10 inhibitor is expected, or a subject who has been determined to have a high risk of exacerbation of the novel coronavirus (SARS-CoV-2) disease 2019 (COVID-19).

12. The pharmaceutical composition according to any of claims 1 to 11, for administration to a subject who has been determined to be a subject of administration, wherein
the above determination is performed based on:
1) measurement of the blood concentration of IL-10 in the subject;
2) measurement of the expression level of the IL-10 receptor in the subject;
3) quantification of chimeric transcripts of an IL10RB gene and an IFNAR2 gene;
4) measurement of the expression level of the protein (CIDRE) having all or a part of the amino acid sequence of the interferon-α receptor 2 and all or a part of the amino acid sequence of the IL-10 receptor β; or
5) presence or absence of mutations in a genomic region that controls alternative splicing of the IL10RB gene and/or the IFNAR2 gene.

13. The pharmaceutical composition according to claim 12, wherein the subject is determined to be a subject of administration when the blood concentration of IL-10 in the subject is equal to or more than the predetermined value.

14. The pharmaceutical composition according to claim 12, wherein the subject is determined to be a subject of administration when the expression level of the IL-10 receptor in the subject is equal to or more than the predetermined level.

15. The pharmaceutical composition according to claim 12, wherein the subject is determined to be a subject of administration when an amount of chimeric transcripts of the IL10RB gene and the IFNAR2 gene in the subject is equal to or more than the predetermined amount.

16. The pharmaceutical composition according to claim 12, wherein the subject is determined to be a subject of administration when the expression level of CIDRE is equal to or more than the predetermined level.

17. The pharmaceutical composition according to claim 12, wherein the subject is determined to be a subject of administration when a mutation is present in the genomic region that controls alternative splicing of the IL10RB gene.

18. The pharmaceutical composition according to claim 12, wherein the subject is determined to be a subject of administration when a mutation is present at rs13050728.

19. The pharmaceutical composition according to any one of claims 1 to 18, wherein the subject has a past history of lung disease.

20. A method for determining risk of exacerbation of the novel coronavirus (SARS-CoV-2) disease 2019 (COVID-19), in which
the above determination comprises:
1) measurement of the blood concentration of IL-10 in the subject;
2) measurement of the expression level of the IL-10 receptor in the subject;
3) quantification of chimeric transcripts of an IL10RB gene and an IFNAR2 gene;
4) measurement of the expression level of the protein (CIDRE) having all or a part of the amino acid sequence of the interferon-α receptor 2 and all or a part of the amino acid sequence of the IL-10 receptor β; or
5) confirmation of the presence or absence of mutations in a genomic region that controls alternative splicing of the IL10RB gene and/or the IFNAR2 gene.

21. The method according to claim 20, further comprising determining that the subject has a high risk of the exacerbation when the blood concentration of IL-10 in the subject is equal to or more than the predetermined value.

22. The method according to claim 20, further comprising determining that the subject has a high risk of the exacerbation when the expression level of the IL-10 receptor in the subject is equal to or more than the predetermined level.

23. The method according to claim 20, further comprising determining that the subject has a high risk of the exacerbation when the amount of chimeric transcripts of the IL10RB gene and the IFNAR2 gene are equal to or more than the predetermined amount.

24. The method according to claim 20, further comprising determining that the subject has a high risk of the exacerbation when the expression level of CIDRE is equal to or more than the predetermined level.

25. The method according to claim 20, further comprising determining that a mutation is present in the genomic region that controls alternative splicing of the IL10RB gene, and the chimeric transcripts of the IL10RB gene and the IFNAR2 gene are detected in the subject.

26. The method according to claim 20, further comprising determining that a mutation is present at rs13050728, and the chimeric transcripts of the IL10RB gene and the IFNAR2 gene are detected in the subject.

27. The method according to any one of claims 20 to 26, wherein the determination is performed using a blood sample collected from the subject.

28. The method according to any one of claims 20 to 27, wherein the subject is not infected with SARS-CoV-2.
